(19) Europäisches Patentamt / European Patent Office / Office européen des brevets

(11) **EP 4 696 297 A1**

(12) **EUROPEAN PATENT APPLICATION**
published in accordance with Art. 153(4) EPC

(43) Date of publication:
**18.02.2026   Bulletin 2026/08**

(21) Application number: **24788839.9**

(22) Date of filing: **12.04.2024**

(51) International Patent Classification (IPC):
*A61K 6/889* (2020.01)    *A61K 6/15* (2020.01)
*A61K 6/802* (2020.01)    *A61K 6/836* (2020.01)
*A61K 6/887* (2020.01)

(52) Cooperative Patent Classification (CPC):
A61K 6/15; A61K 6/802; A61K 6/836; A61K 6/887;
A61K 6/889

(86) International application number:
**PCT/JP2024/014840**

(87) International publication number:
**WO 2024/214817 (17.10.2024 Gazette 2024/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.04.2023   JP 2023066013**

(71) Applicant: **Yamakin Co.,Ltd.
Konan-shi, Kochi 781-5451 (JP)**

(72) Inventors:
• **IWAMOTO Kouki
  Konan-shi, Kochi 781-5451 (JP)**
• **KATO Takahiro
  Konan-shi, Kochi 781-5451 (JP)**
• **NAKAGOSHI Shuji
  Konan-shi, Kochi 781-5451 (JP)**
• **SATO Yuji
  Konan-shi, Kochi 781-5451 (JP)**

(74) Representative: **Hasegawa, Kan
Patentanwaltskanzlei Hasegawa
Untere Hauptstraße 56
85354 Freising (DE)**

(54) **COMPOSITE MATERIAL FOR DENTAL CUTTING**

(57)    The problem to be solved by the present invention is to provide a composite material for dental milling from which a dental prosthesis having a three-point bending strength required for a dental bridge and a three-point bending elastic modulus similar to those of tooth substances can be produced by a single milling operation using a CAD/CAM system. The present invention relates to a composite material for dental milling comprising (A) a first member and (B) a second member in contact with at least a portion of the first member (A), wherein the first member (A) comprises (C) a first curable resin and (D) a glass fiber, wherein the second member (B) comprises (E) a second curable resin and (F) an inorganic filler, wherein the first member (A) has a three-point bending strength of 500 MPa or more, and wherein the second member (B) has a three-point bending strength of less than 500 MPa.

FIG.1

**Description**

Technical Field

The present invention relates to a composite material for dental milling.

Background Art

In the case of tooth loss, there are currently three main options of treatment methods, namely, bridges, dentures, and implants, to replace the missing teeth. Of these, a bridge is formed by an abutment portion, a pontic portion, and a connecting portion as an integral piece. The abutment portion is a portion that uses a tooth (remaining tooth) that remains on one or both sides of a missing tooth as an abutment tooth (base), in order to fix an artificial tooth for replacing the missing tooth to the abutment tooth. The pontic portion is a portion for artificially restoring the function and form of the missing tooth. The connecting portion is a portion for connecting the abutment portion and pontic portion. When such a bridge is used as, for example, a three-teeth connected prosthesis (three-unit bridge) including a molar tooth, it supports an artificial tooth for replacing a missing tooth by using the neighboring teeth on both sides as abutment teeth, and therefore requires a certain degree of strength. For example, a three-unit bridge is required to have a bending strength of 500 MPa or more as measured by a three-point bending test in accordance with JIS T 6526 (Dental ceramic materials).

As dental materials for use in bridges, for example, fiber reinforced plastics (FRPs), which are composites of resins (plastics) with fibers, have been previously proposed (Patent Literature 1).

Patent Literature 1 discloses a dental material comprising a fiber impregnated with a polymerizable monomer composition containing a (meth)acrylate-based monomer composition, a polymerization initiator, and an inorganic powder. A dental prosthesis such as a bridge can be produced using such a dental material obtained from a resin; however, the production of a dental prosthesis needs to be performed manually by a dental technician. Furthermore, the production method disclosed in Patent Literature 1 requires many working steps and is complicated. Therefore, according to the production method disclosed in Patent Literature 1, the production of a dental prosthesis is time-consuming, and the quality may depend on the skill of the dental technician.

Therefore, in recent years, dental CAD/CAM (Computer-Aided Design/Computer-Aided Manufacturing) systems have become widespread for the purpose of labor saving, for example. Use of these systems have enabled the production of various dental prostheses. For example, a frame portion of a dental prosthesis can be milled from a cured product of a fiber-reinforced plastic, and a crown portion can be produced by manually building up a resin material on the frame portion.

However, these systems involve a complicated production process, and if air bubbles are included in the resin material, the strength of the dental prosthesis may be reduced.

Furthermore, a crown portion of a dental prosthesis can be produced from a cured product of a dental resin by using a CAD/CAM system.

However, the crown portion and a frame portion separately produced using the CAD/CAM system need to be manually formed into an integral piece, using a dental adhesive resin cement. This has problems in terms of production, for example, if air bubbles are included in the dental adhesive resin cement, the strength of the dental prosthesis may be reduced.

Patent Literature 2 discloses a block-like composite material for dental cutting and processing, wherein the block-like composite material has a multilayered structure comprising at least two layers having different transparencies, wherein each of the layers includes a curable resin and a fiber material. The block-like composite material can be cut and processed using a CAD/CAM system to produce a dental prosthesis device.

However, only the contrast ratio was evaluated in the examples of Patent Literature 2, and the mechanical properties of the block-like composite material for dental cutting and processing are unknown.

In addition, the block-like composite material for dental cutting and processing disclosed in Patent Literature 2 does not include a layer of a dental resin not containing a fiber material. Therefore, when using a dental prosthesis device that is produced by cutting and processing the block-like composite material for dental cutting and processing, without subsequently building up a dental resin not containing a fiber material thereon, the fiber material may be exposed in the oral cavity due to abrasion caused by occlusion in the oral cavity, possibly resulting in an injury in the oral cavity.

On the other hand, not only the dental material obtained from a fiber-reinforced plastic in which a resin is composited with a fiber as disclosed in Patent Literature 1, but also zirconia, which is a dental ceramic material, is used to produce dental bridges. Since zirconia has high material strength, the use of zirconia eliminates the need to provide a frame portion for reinforcement, and allows an entire dental prosthesis to be produced by milling.

However, since the milled product produced from zirconia is a semi-sintered body, it is necessary to perform a permanent sintering step in which the semi-sintered body is fired at a high temperature after milling. Therefore, dental bridges produced from zirconia also have a problem in terms of production, namely that the production process is complicated.

Therefore, there is a need for a composite material for dental milling from which a dental prosthesis for a bridge can be produced by only a single milling operation and from which a dental prosthesis having a three-point bending strength required for a dental bridge and a three-point bending elastic modulus similar to those of tooth substances can be produced.

Citation List

Patent Literature

Patent Literature 1: JP-A-2003-104822
Patent Literature 2: JP-A-2017-124981

Summary of Invention

Technical Problem

It is an object of the present invention to provide a composite material for dental milling from which a dental prosthesis having a three-point bending strength required for a dental bridge and a three-point bending elastic modulus similar to those of tooth substances can be produced by a single milling operation using a CAD/CAM system.

Solution to Problem

The inventors of the present invention have conducted extensive research to find that a composite material for dental milling in which at least a portion of a first member comprising a curable resin and a glass fiber and having a three-point bending strength of 500 MPa or more is in contact with a second member comprising a curable resin and an inorganic filler and having a three-point bending strength of less than 500 MPa can achieve the above-mentioned object, thus completing the present invention.

In summary, the present invention is as itemized below:

Item 1. A composite material for dental milling comprising (A) a first member and (B) a second member in contact with at least a portion of the first member (A),

wherein the first member (A) comprises (C) a first curable resin and (D) a glass fiber,
wherein the second member (B) comprises (E) a second curable resin and (F) an inorganic filler,
wherein the first member (A) has a three-point bending strength of 500 MPa or more, and
wherein the second member (B) has a three-point bending strength of less than 500 MPa.

Item 2. The composite material for dental milling according to item 1, wherein the second member (B) is coated or laminated on at least a portion of the first member (A).
Item 3. The composite material for dental milling according to item 1, wherein a ratio of a cross-sectional area of the second member (B) to a cross-sectional area of the first member (A) is 0.05 to 100.
Item 4. The composite material for dental milling according to item 1, wherein the cross-sectional area of the first member (A) is 3 to 330 mm$^2$.
Item 5. The composite material for dental milling according to item 1, wherein the cross-sectional area of the second member (B) is 270 to 330 mm$^2$.
Item 6. The composite material for dental milling according to item 1, wherein the first member (A) further comprises a coloring pigment.
Item 7. The composite material for dental milling according to item 1, wherein the composite material for dental milling has a three-point bending elastic modulus of 14 to 33 GPa.
Item 8. A dental prosthesis produced by milling from the composite material for dental milling according to any one of items 1 to 7.
Item 9. The dental prosthesis according to item 8, wherein the dental prosthesis is a bridge.
Item 10. The dental prosthesis according to item 9, wherein the bridge comprises an abutment portion, a connecting portion, and a pontic portion.
Item 11. The dental prosthesis according to item 10, wherein the dental prosthesis has a fracture strength of 3000 N or more.

Item 12. The composite material for dental milling according to item 1, wherein the first member (A) has a volume of 100 to 20000 mm$^3$.

Item 13. The composite material for dental milling according to item 1, wherein the second member (B) has a volume of 20 to 11000 mm$^3$.

Item 14. A composite material for dental milling comprising (A1) a core material and (B1) a coating material that coats at least a portion of the core material (A1),

> wherein the core material (A1) comprises (C) a first curable resin and (D) a glass fiber,
> wherein the coating material (B1) comprises (E) a second curable resin and (F) an inorganic filler,
> wherein the core material (A1) has a three-point bending strength of 500 MPa or more,
> wherein the coating material (B1) has a three-point bending strength of less than 500 MPa, and
> wherein the core material (A1) has a cross-sectional area of 3 to 14 mm$^2$.

Item 15. The composite material for dental milling according to item 14, wherein the core material (A1) has a volume of 100 to 460 mm$^3$.

Item 16. The composite material for dental milling according to item 14, wherein the core material (A1) further comprises a coloring pigment.

Item 17. The composite material for dental milling according to item 14, wherein the composite material for dental milling has a three-point bending elastic modulus of 14 to 33 GPa.

Item 18. The composite material for dental milling according to item 14, wherein the composite material for dental milling is used for a bridge.

Item 19. A dental prosthesis produced by milling from the composite material for dental milling according to any one of items 14 to 18.

Item 20. The dental prosthesis according to item 19, wherein the dental prosthesis is a bridge.

Item 21. The dental prosthesis according to item 20, wherein the bridge comprises an abutment portion, a connecting portion, and a pontic portion.

Item 22. The dental prosthesis according to item 21, wherein the dental prosthesis has a fracture strength of 3000 N or more.

At present, it is impossible or impractically difficult to completely specify the structure of or the whole range of components contained in the composite material for dental milling as defined by the production process, among the aspects of the present invention, and therefore, the composite material for dental milling is specified in a product-by-process claim.

Advantageous Effects of Invention

According to the present invention, it is possible to provide a composite material for dental milling from which a dental prosthesis having a three-point bending strength required for a dental bridge and a three-point bending elastic modulus similar to those of tooth substances can be produced by a single milling operation using a CAD/CAM system.

Furthermore, the composite material for dental milling has a density similar to those of tooth substances, so that a dental prosthesis produced from the composite material for dental milling is less likely to cause loss of occlusal balance when placed in the oral cavity, and therefore, can be expected to reduce health hazards (such as abrasion or fracture of teeth, temporomandibular disorders, stiff shoulders, and stress) caused by loss of occlusal balance.

Brief Description of Drawings

FIG. 1 is a perspective view showing one example of the composite material for dental milling according to the present invention.

FIG. 2 is a perspective view showing another example of the composite material for dental milling according to the present invention.

FIG. 3a is a front view of the other example of the composite material for dental milling according to the present invention, and FIG. 3b is a cross-sectional view along the line A-A' of the front view.

FIG. 4 is a schematic diagram for explaining a three-unit bridge fracture test.

FIG. 5 is a cross-sectional schematic view showing an example of the dental prosthesis (three-unit bridge) of the present invention.

FIG. 6 is a cross-sectional schematic view showing another example of the dental prosthesis (three-unit bridge) of the present invention.

FIG. 7 is a perspective view showing a specimen of Example 20 used in the three-unit bridge fracture test, wherein the two portions shown with a dashed line in FIG. 7 indicate connecting portions of the three-unit bridge.

FIG. 8 is a cross-sectional schematic view of the specimen of Example 20 used in the three-unit bridge fracture test shown in FIG. 7.

FIG. 9 is a cross-sectional schematic view of a specimen of Comparative Example 8 used in the three-unit bridge fracture test.

Description of Embodiments

Composite Material for Dental Milling

The composite material for dental milling of the present invention is a composite material for dental milling comprising (A) a first member and (B) a second member in contact with at least a portion of the first member (A),

wherein the first member (A) comprises (C) a first curable resin and (D) a glass fiber, wherein the second member (B) comprises (E) a second curable resin and (F) an inorganic filler,
wherein the first member (A) has a three-point bending strength of 500 MPa or more, and
wherein the second member (B) has a three-point bending strength of less than 500 MPa.

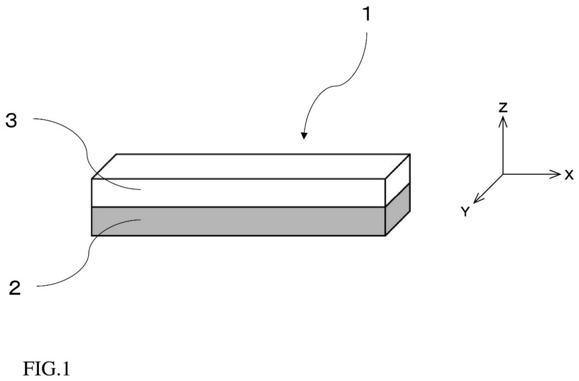

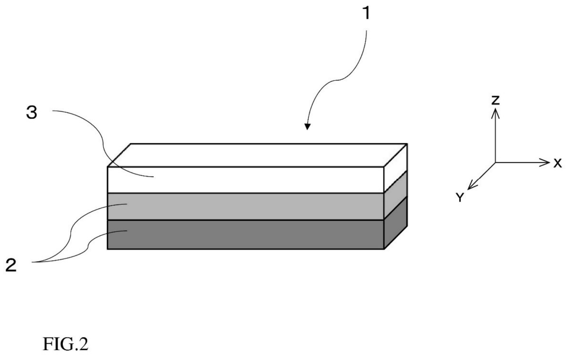

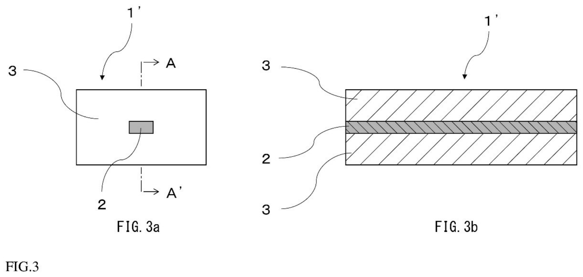

The composite material for dental milling of the present invention is not limited to specific types, and may be, for example, of a coated core material type (which can also be referred to as a core-sheath structure type, a core-shell type, or the like) as shown in FIG. 3, or of a laminate type (which can also be referred to as a laminate) as shown in FIGS. 1 and 2.

(A) First Member

The first member (A) comprises (C) a first curable resin and (D) a glass fiber. The first member (A) can also be referred to as "first member", "(A) first member", "core material", "(A1) core material" or "core material (A1)".

When the composite material for dental milling of the present invention is of the coated core material type as shown in FIG. 3, the composite material includes (A1) a core material and (B1) a coating material that coats at least a portion of the core material (A1).

When the composite material for dental milling of the present invention is of the laminate type (two-layer type) as shown in FIG. 1, the composite material includes (A2) a first layer and (B2) a second layer. Furthermore, when the composite material for dental milling of the present invention is of the laminate type (three-layer type) as shown in FIG. 2, the composite material includes (A2-1) a first-first layer, (A2-2) a first-second layer, and (B2) a second layer.

The core material (A1) includes (C) a first curable resin and (D) a glass fiber.

(C) First Curable Resin

The first curable resin (C) may be, for example, a product obtained by polymerizing and curing a polymerizable monomer-containing composition (hereinafter, sometimes also referred to as "first polymerizable monomer-containing composition") comprising:

(C1) a polyfunctional (meth)acrylate-based polymerizable monomer; and/or
(C2) a low-viscosity polymerizable monomer.

That is, the first member (A) may be, for example, a product obtained by polymerizing and curing a first member composition comprising:

a polymerizable monomer-containing composition comprising:

(C1) a polyfunctional (meth)acrylate-based polymerizable monomer; and
(C2) a low-viscosity polymerizable monomer; and (D) a glass fiber.

Since the first member composition contains the glass fiber (D), the first member, which is a cured product of the first member composition, contains the glass fiber (D) with the first curable resin (C).

(C1) Polyfunctional (Meth)Acrylate-Based Polymerizable Monomer

In the present invention, the polyfunctional (meth)acrylate-based polymerizable monomer (C1) (hereinafter, sometimes also referred to as "component C1") is used as a polymerizable monomer constituting a structural unit of the first curable resin (C) of the first member (A). As used herein, the term (meth)acrylate refers to acrylate or methacrylate. The polyfunctional (meth)acrylate-based polymerizable monomer (C1) is a monomer used for the purpose of imparting high strength to the composite material for dental milling.

The polyfunctional (meth)acrylate-based polymerizable monomer (C1) may be a polyfunctional (meth)acrylate-based polymerizable monomer that can be used for dental applications and has a viscosity at 31°C of more than 5 mPa•s. Examples of such polyfunctional (meth)acrylate-based polymerizable monomers include di(meth)acrylates such as di(meth)acrylates containing bisphenol A, and a urethane di(meth)acrylate, which is a reaction product between 2 moles of a (meth)acrylate having a hydroxyl group and 1 mole of a diisocyanate; tri(meth)acrylates such as trimethylolpropane tri(meth)acrylate; and tetra(meth)acrylates such as pentaerythritol tetra(meth)acrylate.

Examples of the polyfunctional (meth)acrylate (C1) include di(meth)acrylates such as bisphenol A di(meth) acrylate, ethoxylated bisphenol A di(meth)acrylate, ethylene oxide-modified bisphenol A glycidyl di(meth)acrylate, 2,2-bis(4-methacryloxypropoxyphenyl)propane, 7,7,9-trimethyl-4,13-dioxa-3,14-dioxo-5,12-diazahexadecane-1,16-diol di(meth)acrylate, neopentyl glycol hydroxypivalic acid ester di(meth)acrylate, caprolactone-modified hydroxypivalic acid neopentyl glycol ester di(meth)acrylate, trimethylol ethane di(meth)acrylate, trimethylol propane di(meth)acrylate, urethane di(meth)acrylate (1,6-bis((meth)acryloyloxy-2-ethoxycarbonylamino)-2,4,4-trimethylhexane), the reaction product of 3-chloro-2-hydroxypropyl (meth)acrylate and methylcyclohexane diisocyanate, the reaction product of 2-hydroxypropyl (meth)acrylate and methylcyclohexane diisocyanate, the reaction product of 2-hydroxypropyl (meth)acrylate and methylenebis (4-cyclohexylisocyanate), the reaction product of 2-hydroxypropyl (meth)acrylate and trimethyl hexamethylene diisocyanate, the reaction product of 2-hydroxyethyl (meth)acrylate and isophorone diisocyanate, and the reaction product of 3-chloro-2-hydroxypropyl (meth)acrylate and isophorone diisocyanate; tri(meth)acrylates such as trimethylolmethane tri(meth)acrylate, trimethylolethane tri(meth)acrylate, trimethylolpropane tri(meth)acrylate, pentaerythritol tri(meth)acrylate, and dipentaerythritol tri(meth)acrylate; and tetra(meth)acrylates such as pentaerythritol tetra(meth)acrylate and dipentaerythritol tetra(meth)acrylate.

Preferred as the polyfunctional (meth)acrylate-based polymerizable monomer (C1) are trimethylolpropane trimethacrylate (TMPTMA), urethane dimethacrylate (UDMA), bisphenol A glycidyl dimethacrylate (Bis-GMA), and ethoxylated bisphenol A dimethacrylate (Bis-EMA), and more preferred are UDMA and Bis-EMA.

These polyfunctional (meth)acrylate-based polymerizable monomers (C1) can be used alone, but may be used in combinations of two or more.

The first curable resin (C) contained in the first member (A) may be obtained by polymerizing a mixture of the polyfunctional (meth)acrylate-based polymerizable monomer (C1) with other polymerizable monomers besides the polyfunctional (meth)acrylate-based polymerizable monomer, for example, the below-described low-viscosity polymerizable monomer (C2), for regulating the viscosity, polymerizability, or other physical properties.

(C2) Low-Viscosity Polymerizable Monomer

The low-viscosity polymerizable monomer (C2) is a monomer used for the purpose of adjusting the viscosity of the polymerizable monomer-containing composition to be impregnated into the glass fiber to improve the impregnation. The low-viscosity polymerizable monomer (C2) is a monomer that serves as a diluent for the polyfunctional (meth) acrylate-based polymerizable monomer (C1), that is, has the effect of reducing the viscosity, and that is miscible with the polyfunctional (meth)acrylate-based polymerizable monomer (C1). The low-viscosity polymerizable monomer (C2) does not include the polyfunctional (meth)acrylate-based polymerizable monomer (C1).

The low-viscosity polymerizable monomer (C2) may be a polymerizable monomer having a low viscosity, for example, a viscosity of 5 mPa•s or less at 31°C. Examples of the low-viscosity polymerizable monomer (C2) include methacrylic acid (viscosity: less than 0.3 mPa•s (31°C), less than 1.34 mPa•s (25°C)), diethylene glycol dimethacrylate (DEGDMA) (viscosity: 3 mPa•s (31°C), less than 5 mPa•s (25°C)), triethylene glycol dimethacrylate (TEGDMA) (viscosity: 5 mPa•s (31°C), less than 9 mPa•s (25°C)), acrylic acid (viscosity: 1.3 mPa•s (20°C)), methyl acrylate (viscosity: 0.482 mPa•s (21°C)), ethyl acrylate (viscosity: 0.535 mPa•s (25°C)), 1,6-hexanediol diacrylate (viscosity: 6 mPa•s (25°C)), neopentyl glycol diacrylate (viscosity: 6 mPa•s (25°C)), methyl methacrylate (viscosity: 0.56 mPa•s (20°C)), ethyl methacrylate (viscosity: 1 mPa•s or less (25°C)), methoxydiethylene glycol methacrylate (viscosity: 2 mPa•s (25°C)), methoxytetraethylene glycol methacrylate (viscosity: 7 mPa•s (25°C)), phenoxyethylene glycol methacrylate (viscosity: 7 mPa•s (25°C)), ethylene glycol dimethacrylate (viscosity: 3.2 mPa•s (20°C)), 1,6-hexanediol dimethacrylate (viscosity: 6 mPa•s (25°C)), and neopentyl glycol dimethacrylate (viscosity: 5 mPa•s (25°C)). Because of their short molecular length, these polymerizable monomers also have the effect of increasing the polymerization rate during curing. The lower limit of the viscosity is not specifically limited and may be, for example, about 0.01 mPa•s.

Preferred as the low-viscosity polymerizable monomer (C2) are methacrylic acid, diethylene glycol dimethacrylate, and triethylene glycol dimethacrylate.

The first polymerizable monomer-containing composition is only required to contain either one of the polyfunctional (meth)acrylate-based polymerizable monomer (C1) and the low-viscosity polymerizable monomer (C2); and the first polymerizable monomer-containing composition may contain the polyfunctional (meth)acrylate-based polymerizable monomer (C1) and the low-viscosity polymerizable monomer (C2). The first polymerizable monomer-containing composition preferably contains the polyfunctional (meth)acrylate-based polymerizable monomer (C1) and the low-viscosity polymerizable monomer (C2).

The content of the polyfunctional (meth)acrylate-based polymerizable monomer (C1) in the first polymerizable monomer-containing composition is typically 0 to 100% by mass, preferably 10 to 90% by mass, and more preferably 20 to 80% by mass.

The content of the low-viscosity polymerizable monomer (C2) in the first polymerizable monomer-containing composition is typically 0 to 100% by mass, preferably 10 to 50% by mass, and more preferably 20 to 40% by mass.

When the first polymerizable monomer-containing composition contains the polyfunctional (meth)acrylate-based polymerizable monomer (C1) and the low-viscosity polymerizable monomer (C2), the content of the low-viscosity polymerizable monomer (C2) in the first polymerizable monomer-containing composition is typically 5 to 150 parts by mass, preferably 10 to 100 parts by mass, more preferably 20 to 70 parts by mass, and still more preferably 30 to 50 parts by mass, per 100 parts by mass of the polyfunctional (meth)acrylate-based polymerizable monomer (C1).

A polymerizable monomer mixture containing the polyfunctional (meth)acrylate-based polymerizable monomer (C1) and the low-viscosity polymerizable monomer (C2) has a viscosity at 31°C of typically 8 to 2300 mPa•s, preferably 15 to 1000 mPa•s, and more preferably 25 to 150 mPa•s. Setting the viscosity of the polymerizable monomer mixture within the above-mentioned ranges can prevent molding defects due to significant shrinkage of the polymerizable monomer-containing composition during polymerization and curing, and allows the polymerizable monomer-containing composition to be sufficiently impregnated between the glass fibers, so that the first member (core material) having high strength can be obtained.

The viscosity of each of the polyfunctional (meth)acrylate-based polymerizable monomer (C1), the low-viscosity polymerizable monomer (C2), and the polymerizable monomer mixture can be determined by measuring the viscosity at 31°C using an SV-type (tuning-fork vibration-type) viscometer or a B-type (rotational-type) viscometer.

Optional Additives

The composite material for dental milling of the present invention may optionally further contain optional additives.

Examples of additives include a polymerization initiator (G), a polymerization accelerator, a coloring pigment, an opacifying agent, a fluorescent material, an opalizing agent, a polymerization inhibitor, an antioxidant, an antibacterial agent, an X-ray contrast agent, a stabilizer, an ultraviolet absorber, a discoloration inhibitor, and various other known additives. These additives can be used alone or in appropriate combinations of two or more. The polymerizable monomer-containing composition preferably contains a polymerization initiator (G) in view of production efficiency, and more preferably contains a polymerization initiator (G) and a coloring pigment in view of production efficiency and aesthetics.

The polymerization initiator (G) is not specifically limited as long as it is a commonly used polymerization initiator, and is particularly preferably a polymerization initiator used for dental applications. In general, different types of polymerization initiators are used depending on the polymerization means for the polymerizable monomer. For example, when the polymerization means is photoirradiation, a photopolymerization initiator is used. When the polymerization means is heat treatment, a thermal polymerization initiator is used.

The photopolymerization initiator is not specifically limited, and may be a photopolymerization initiator that reacts with ultraviolet or visible light to generate radicals. Specific examples include $\alpha$-diketones such as diacetyl, acetylbenzoyl, benzyl, camphorquinone (CQ), 9,10-phenanthrenequinone, and acenaphthenequinone; benzoin alkyl ethers such as benzoin methyl ether, benzoin ethyl ether, and benzoin propyl ether; thioxanthone compounds such as 2,4-diethylthioxanthone, 2,4,6-trimethylbenzoyl diphenylphosphine oxide, and methylthioxanthone; and benzophenone-based compounds such as benzophenone, p,p'-dimethylaminobenzophenone, and p,p'-dimethoxyaminobenzophenone.

The thermal polymerization initiator is not specifically limited, and may be a known thermal polymerization initiator, such as a peroxide or an azo compound. Specific examples include benzoyl peroxide (BPO), ketone peroxides, peroxyketals, hydroperoxides, dialkyl peroxides, diacyl peroxides, peroxyesters, peroxydicarbonates, 2,2'-azobisisobutyronitrile, 2,2'-azobis-2,4-dimethylvaleronitrile, 4,4'-azobis-4-cyanovaleric acid, 1,1'-azobis-1-cyclohexanecarbonitrile, dimethyl-2,2'-azobisisobutyrate, and 2,2'-azobis-(2-aminopropane) dihydrochloride.

The content of the polymerization initiator (G) is typically 0.002 to 5 parts by mass, preferably 0.01 to 3 parts by mass, more preferably 0.05 to 1 part by mass, and still more preferably 0.1 to 0.3 parts by mass, per total 100 parts by mass of the polyfunctional (meth)acrylate-based polymerizable monomer (C1) and the low-viscosity polymerizable monomer (C2). Setting the content of the polymerization initiator (G) within the above-mentioned ranges allows the polymerization initiator (G) to be dissolved in the polyfunctional (meth)acrylate-based polymerizable monomer (C1) and the low-viscosity polymerizable monomer (C2), and allows the polymerizable monomer-containing composition to be polymerized and cured. Even a very small amount (0.01 parts by mass or less) of the polymerization initiator (G) can sufficiently cure the polymerizable monomer-containing composition to obtain the first member (A) having high strength.

In the polymerization of the polymerizable monomer used for the first member (A), the polymerization initiator (G) described above may be used in combination with a polymerization accelerator.

The polymerization accelerator is generally used in combination with a photopolymerization initiator. Examples of the polymerization accelerator include, but are not specifically limited to, 2-(dimethylamino)ethyl methacrylate (DMAEMA), ethyl 2-(dimethylamino)benzoate methacrylate (DMABE), and n-butoxyethyl 2-(dimethylamino)benzoate methacrylate.

Method for Producing First Polymerizable Monomer-Containing Composition

The first polymerizable monomer-containing composition can be produced by mixing the polyfunctional (meth) acrylate-based polymerizable monomer (C1), the low-viscosity polymerizable monomer (C2), and optionally the polymerization initiator (G).

The proportions (mixing ratio) of the components in the first polymerizable monomer-containing composition can be appropriately adjusted according to the viscosity and the purpose of use. When the polymerization initiator (G) is mixed, the proportion (content) of the polymerization initiator (G) may be, for example, 0.002 to 5 parts by mass per total 100 parts by mass of the polyfunctional (meth)acrylate-based polymerizable monomer (C1) and the low-viscosity polymerizable monomer (C2).

Optionally, the first polymerizable monomer-containing composition may further contain additives such as an inorganic filler, a polymerization accelerator, a coloring pigment, an opacifying agent, an opalizing agent, a fluorescent material, a polymerization inhibitor, an antioxidant, an antibacterial agent, an X-ray contrast agent, a stabilizer, an ultraviolet absorber, and a discoloration inhibitor, as appropriate.

The method for producing the first polymerizable monomer-containing composition includes the step of adding a predetermined amount of each of the components described above to a container and sufficiently kneading the components to give a uniform dispersion.

(D) Glass Fiber

The first member (A) comprises a glass fiber (D). As the glass fiber (D), any known glass fibers used as dental materials can be used without limitation.

Examples of materials of the glass fiber (D) include A-glass, C-glass, D-glass, E-glass, ECR-glass, AR-glass, and S-glass. A-glass refers to a glass composition containing 72% by mass of $SiO_2$, 0.6% by mass of $Al_2O_3$, 10% by mass of CaO, 2.5% by mass of MgO, and 14.7% by mass of $Na_2O$, based on the total amount. C-glass refers to a glass composition containing 65% by mass of $SiO_2$, 14% by mass of $Al_2O_3$, 5% by mass of $B_2O_3$, 7 to 14% by mass of CaO, 3% by mass of MgO, 8 to 11% by mass of $Na_2O$, and 1% by mass of $K_2O$, based on the total amount. D-glass refers to a glass composition containing 74% by mass of $SiO_2$, 0.5% by mass of $Al_2O_3$, 22% by mass of $B_2O_3$, 0.5% by mass of CaO, 1% by mass of $Na_2O$, 1.5% by mass of $K_2O$, and 0.5% by mass of $Li_2O$, based on the total amount. E-glass refers to a glass composition containing 52 to 56% by mass of $SiO_2$, 12 to 16% by mass of $Al_2O_3$, 0 to 0.8% by mass of $Fe_2O_3$, 0 to 13% by mass of $B_2O_3$, 15 to 25% by mass of CaO, 0 to 6% by mass of MgO, 0 to 2% by mass in total of $Na_2O + K_2O$, 0 to 1.5% by mass of $TiO_2$, and 0 to 1% by mass of $F_2$, based on the total amount. ECR-glass refers to a glass composition containing 58.2% by mass of $SiO_2$, 11.3% by mass of $Al_2O_3$, 0.3% by mass of $Fe_2O_3$, 22% by mass of CaO, 2.7% by mass of MgO, 0.1% by mass of $Na_2O$, 0.5% by mass of $K_2O$, 2.2% by mass of $TiO_2$, and 2.7% by mass of ZnO, based on the total amount. AR-glass refers to a glass composition containing 62.5% by mass of $SiO_2$, 0.5% by mass of $Al_2O_3$, 0 to 0.8% by mass of $Fe_2O_3$, 5.7% by mass of CaO, 14.2% by mass of $Na_2O$, 0.3% by mass of $K_2O$, and 16.8% by mass of $ZrO_2$, based on the total amount. S-glass refers to a glass composition containing 65% by mass of $SiO_2$, 25% by mass of $Al_2O_3$, and 10% by mass of MgO, based on the total amount. Preferred materials of the glass fiber (D) are E-glass, AR-glass, and S-glass.

The glass fiber (D) typically has an average fiber diameter of 1 to 100 μm, preferably 2 to 20 μm, more preferably 3 to 15 μm, although not specifically limited thereto as long as it achieves the effects of the present invention. The average fiber diameter can be determined by electron microscopy. Specifically, the average fiber diameter of the glass fiber can be determined by using a scanning electron microscope (SEM; for example, "Model SU3500H-800NA", manufactured by Hitachi High-Technologies Corporation) to take an SEM image of a cross section perpendicular to the orientation direction of the glass fiber, and measuring the fiber diameters of (100 or more) glass fibers observed in the unit field of view of the SEM image, using image analysis-type particle size distribution measuring software (for example, "Mac-View", manufactured by Mountech Co., Ltd.). Here, the fiber diameter of the glass fiber is determined as the equivalent circle diameter that is the diameter of a circle having the same area as the area of the glass fiber, and the average fiber diameter is calculated from the number of the glass fibers and their fiber diameters. The glass fiber (D) may be a commercial product having the above-mentioned average fiber diameter. The average fiber diameter of the glass fiber (D) can be adjusted, for example, by mixing fibers (for example, commercial products) with different average fiber diameters.

The glass fiber (D) is not limited to specific shapes as long as it achieves the effects of the present invention, but is preferably in the form of a woven structure in which glass fibers are woven (hereinafter sometimes also referred to as "glass fiber woven fabric"), and more preferably a straight glass fiber that is not in the form of a woven structure. The woven structure refers to a structure formed by weaving a glass fiber in two directions of the warp and weft to intersect each other, and examples include plain weave, twill weave, satin weave, basket weave, leno weave, and mock leno weave.

The glass fiber (D) may optionally be treated beforehand with a surface treatment agent. The glass fiber (D) is preferably treated with a surface treatment agent, in view of enhancing the adhesion to the curable resin with which it forms a composite, and improving the mechanical strength.

Any known materials and methods may be employed without limitation as the surface treatment agent to be used and the surface treatment method. Examples of the surface treatment agent include silane compounds, for example, compounds used as surface-modifying agents of inorganic oxides, including silane coupling agents, such as 3-(meth) acryloyloxypropyltrimethoxysilane, 4-(meth)acryloyloxybutyltrimethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 12-(meth)acryloyloxydodecyltrimethoxysilane, 13-(meth)acryloyloxytridecyltrimethoxysilane, 14-(meth)acryloyloxytetradecyltrimethoxysilane, vinyltrimethoxysilane, vinyltriethoxysilane, $\gamma$-mercaptopropyltrimethoxysilane, 3-aminopropylethoxysilane, 3-chloropropyltrimethoxysilane silyl isocyanate, $\gamma$-glycidoxypropyltrimethoxysilane, vinyltri($\beta$-methoxyethoxy)silane, and vinyltrichlorosilane.

Methods of treating the glass fiber (D) with a surface treatment agent are not specifically limited, and include, for example, a method in which the glass fiber and the surface treatment agent are heated to reflux in a solvent such as an alcohol for several tens of minutes to about 10 hours, preferably in the range of 1 to 5 hours. If it is necessary to accelerate the hydrolysis of the surface treatment agent, a method may be employed in which water or acidic water such as acetic acid is added to the solvent, and the mixture is heated to reflux for a time in the above-mentioned range, and then the solvent is removed and the resulting product is dried under normal pressure or under reduced pressure.

The proportion of the surface treatment agent is typically in the range of 0.1 to 10 parts by mass, and preferably 0.5 to 5 parts by mass, per 100 parts by mass of the glass fiber (D). The glass fiber after the surface treatment shows almost no change in fiber diameter as compared to the glass fiber before the treatment.

When the glass fiber (D) is a glass fiber woven fabric, the glass fiber woven fabric has a thickness of typically 0.1 to 0.5 mm, and preferably 0.1 to 0.3 mm. The glass fiber woven fabric when in the form of plain weave, for example, has a basis weight of typically 10 to 790 g/m$^2$, and preferably 17 to 400 g/m$^2$.

The glass fiber woven fabric may be a commercial product.

The filling ratio of the glass fiber (D) in the first member (A) is typically 48 to 80% by mass, preferably 60 to 78% by mass, and more preferably 70 to 77% by mass, based on the total amount of the first member (A). Setting the content of the glass fiber (D) in the above-mentioned ranges allows the polymerizable monomer-containing composition to be impregnated into gaps between the glass fibers, so that the first member having sufficient strength can be obtained.

Here, the filling ratio of the glass fiber (D) can be calculated as follows: A specimen of a predetermined size is produced by milling from the first member obtained, and the mass (pre-mass) of the specimen is measured; then, the specimen is heat-treated for a predetermined time using an electric furnace to incinerate the organic components of the first member; and the mass (post-mass) of the residue is measured to calculate the filling ratio. When the glass fiber has been treated with a surface treatment agent, any inorganic component in the surface treatment agent that is not incinerated by the heat treatment is included in the filling ratio of the glass fiber in a strict sense; however, because the amount of the inorganic component contained in the surface treatment agent is negligible, the filling ratio of the glass fiber is substantially unaffected by the inorganic component contained in the surface treatment agent.

In addition to the components (C1) and (C2), and glass fiber (D), the first member (A) may contain the polymerization initiator (G), the various additives described above (such as a polymerization accelerator, a coloring pigment, and an opacifying agent), and the like as long as they do not interfere with the effects of the present invention.

The coloring pigment may be any known material for common dental treatment applications, in order to mimic the color tone or transparency of natural teeth. Some coloring pigments may also serve as an opacifying agent. Examples of the coloring pigment include, but are not specifically limited to, pigments such as inorganic pigments and organic pigments.

Examples of the color types of pigments include a white pigment, a red pigment, a black pigment, and a yellow pigment. Specific examples include iron oxide-based coloring pigments, zirconium oxide pigments, titanium white, and titanium yellow. These coloring pigments can be added singly or in combinations of two or more, preferably in combinations of three or more. For example, ferric oxide (red pigment), isoindolinone (yellow pigment), triiron tetraoxide (black pigment), titanium oxide (white pigment) (all of these manufactured by Dainichiseika Color & Chemicals Mfg. Co., Ltd.), and an opacifying agent (SPZ (zirconium oxide), manufactured by Daiichi Kigenso Kagaku Kogyo Co., Ltd.) can each be added as appropriate.

For example, a blend of coloring pigments in the following proportions can be used as the coloring pigment.

The content of the white pigment is typically 0 to 50000 parts by mass, preferably 1000 to 45000 parts by mass, and more preferably 2000 to 40000 parts by mass, per 100 parts by mass of the red pigment.

The content of the black pigment is typically 0 to 1000 parts by mass, preferably 5 to 900 parts by mass, and more preferably 10 to 800 parts by mass, per 100 parts by mass of the red pigment.

The content of the yellow pigment is typically 0 to 10000 parts by mass, preferably 5 to 9000 parts by mass, and more preferably 10 to 8000 parts by mass, per 100 parts by mass of the red pigment.

When the coloring pigment is added, the coloring pigment is preferably added to the polymerizable monomer-containing composition together with the components (C1) and (C2).

The glass fiber (D) contained in the first member (A) may be not only one type of glass fiber, but also a combination of two or more types of glass fibers. When the glass fiber (D) contains two types of glass fibers, it may be composed of, for example, a glass fiber woven fabric of a glass fiber (D-1) and a glass fiber woven fabric of a glass fiber (D-2). When the glass fiber (D) contains three or more types of glass fibers, it may be composed of, for example, the glass fiber woven fabric of the glass fiber (D-1), the glass fiber woven fabric of the glass fiber (D-2), a glass fiber woven fabric of a glass fiber (D-3), and the like. The glass fiber woven fabrics of the glass fiber (D-1), glass fiber (D-2), glass fiber (D-3), and the like may have different single fiber diameters, thicknesses, and basis weights, for example, and may be laminated as a single layer of each type or as a plurality of layers of each type.

Therefore, when the first member (A) contains two types of glass fibers, the first member (A) may be composed of, for example, a first member (A-1) including the glass fiber woven fabric of the glass fiber (D-1) and a first member (A-2) including the glass fiber woven fabric of the glass fiber (D-2). Alternatively, when the first member (A) contains three or more types of glass fibers, the first member (A) may be composed of, for example, the first member (A-1) including the glass fiber woven fabric of the glass fiber (D-1), the first member (A-2) including the glass fiber woven fabric of the glass fiber (D-2), a first member (A-3) including the glass fiber woven fabric of the glass fiber (D-3), and the like.

Method for Producing First Member

Methods for producing the first member (A) are not specifically limited, and may include exemplary methods described below.

For example, the first member (A) can be produced by mixing the polyfunctional (meth)acrylate-based polymerizable monomer (C1), the low-viscosity polymerizable monomer (C2), and optionally the polymerization initiator (G) to prepare a polymerizable monomer-containing composition, and then impregnating the polymerizable monomer-containing composition into the glass fiber (D), followed by polymerization and curing.

Mixing of the polyfunctional (meth)acrylate-based polymerizable monomer (C1), the low-viscosity polymerizable monomer (C2), and optionally the polymerization initiator (G) may be performed using a common mixer such as a stirrer, a disperser, or a kneader.

Methods of impregnating the glass fiber (D) with the polymerizable monomer-containing composition include various methods typically employed as methods for producing fiber-reinforced plastics (FRPs), for example, (1) a method in which the glass fiber is placed beforehand in a mold having a desired shape, the polymerizable monomer-containing composition is poured onto the glass fiber, and the polymerizable monomer-containing composition is impregnated between the glass fibers through osmotic forces (for example, the capillary action); (2) a method in which the glass fiber is immersed in the polymerizable monomer-containing composition, and then a pressure or reduced pressure is applied thereto, or the polymerizable monomer-containing composition is vigorously sprayed onto the glass fiber, which reduces the time for the polymerizable monomer-containing composition to blend with the glass fiber to accomplish the impregnation in a short time; (3) a method in which the polymerizable monomer-containing composition is heated to reduce the viscosity before being impregnated into the glass fiber; and (4) a method in which the polymerizable monomer-containing composition is degassed by vacuum suction under reduced pressure, while being impregnated into the glass fiber.

When the glass fiber (D) is in the form of a woven fabric structure, for example, when two or more sheets of a glass fiber woven fabric are laminated, a method may be employed, for example, in which a mold having a desired shape is prepared, a first sheet of the glass fiber woven fabric is placed first in the mold, the polymerizable monomer-containing composition is poured into the mold to be impregnated into the glass fiber woven fabric, a second sheet of the glass fiber woven fabric is placed in the mold, and then the polymerizable monomer-containing composition is poured into the mold, so that the sheets of the glass fiber woven fabric are impregnated one by one with the polymerizable monomer-containing composition. When three or more sheets of a glass fiber woven fabric are laminated, the above-described process may be repeated as many times as desired. Alternatively, a method may be employed, for example, in which a plurality of sheets of a glass fiber woven fabric are placed in a mold, and the polymerizable monomer-containing composition is poured into the mold, so that the sheets of the glass fiber woven fabric are impregnated all at once with the polymerizable monomer-containing composition.

That is, when two or more layers of a glass fiber woven fabric are laminated and impregnated with the polymerizable monomer-containing composition, the layers of the glass fiber can be impregnated either one by one or all at once.

When the glass fiber (D) is in the form of a woven fabric structure, and when using the method (1) described above, the direction in which the polymerizable monomer-containing composition is poured may be either parallel or perpendicular to the thickness direction of the glass fiber woven fabric (which is also referred to as "lamination direction" when a plurality of sheets of a glass fiber woven fabric are laminated).

As used herein, "parallel" refers to, for example, the Z-direction shown in FIGS. 1 and 2. "Perpendicular" refers to, for example, the X- or Y-direction shown in FIGS. 1 and 2.

That is, when the direction in which the polymerizable monomer-containing composition is poured is parallel, specifically, for example, layers of a glass fiber woven fabric with a size of 100 mm per side may be laminated and placed in a cavity portion (120 mm in length × 110 mm in width × 10 mm in thickness) of a mold, and the polymerizable monomer-containing composition may be poured in the direction parallel to the lamination direction of (i.e., from above) the glass fiber woven fabric. Then, the polymerizable monomer-containing composition is impregnated into the glass fiber woven fabric and vacuum degassed for 1 to 3 hours in a vacuum oven under vacuum. Then, the polymerizable monomer-containing composition is preferably polymerized by being maintained in a convection oven at 90 to 160°C for 1 to 6 hours.

Alternatively, when the direction in which the polymerizable monomer-containing composition is poured is perpendicular, specifically, for example, layers of a glass fiber woven fabric with a size of 100 mm per side may be laminated and placed in a cavity portion (120 mm in length × 110 mm in width × 10 mm in thickness) of a mold, and the mold may be stood upright so that the lamination surfaces of the glass fiber woven fabric face side surfaces; then the polymerizable monomer-containing composition may be poured in the direction perpendicular to the lamination direction (i.e., in the cross-sectional direction) of the glass fiber woven fabric. Then, the polymerizable monomer-containing composition may be impregnated into the glass fiber woven fabric and vacuum degassed for 1 to 3 hours in a vacuum oven under vacuum. Then, the polymerizable monomer-containing composition is preferably polymerized by being maintained in a convection oven at 90 to 160°C for 1 to 6 hours.

In view of the impregnation and degassing efficiency, for example, of the polymerizable monomer-containing composition, the polymerizable monomer-containing composition is preferably poured in the direction perpendicular to the thickness direction of the glass fiber woven fabric. That is, the efficiency of the impregnation and vacuum degassing is improved by pouring the polymerizable monomer-containing composition in the direction perpendicular to the lamination direction of the glass fiber woven fabric, followed by vacuum degassing. As a result, the first member can be obtained as a uniform composite that is free of air inside.

When performing molding in the mold, a mold and optionally a lid are prepared, for example. The mold has a cavity portion, which is formed in the shape of, for example, a prism, a cylinder, a rectangular plate, or a disc, for example. The mold may be of an assembly type.

Furthermore, when the glass fiber is in the form of a woven fabric structure, and when a plurality of sheets of a glass fiber woven fabric are placed in the cavity portion of the mold, the sheets of the glass fiber woven fabric are preferably placed parallel to the lamination direction thereof, in view of improving the filling ratio of the glass fiber woven fabric.

The means of polymerization and curing is not specifically limited, and examples include heat treatment and/or photoirradiation (photoirradiation and heat treatment, heat treatment alone, or photoirradiation alone). When polymerization and curing is performed by photo irradiation, heat treatment is preferably performed after photo irradiation.

When the means of polymerization and curing is heat treatment, for example, the cavity portion of a mold (a metal, resin, or the like) is filled with the glass fiber woven fabric and the polymerizable monomer-containing composition, then optionally, the mold is covered with a lid, and heat treatment is performed under normal pressure or under pressure to allow the polymerizable monomer-containing composition to be polymerized and cured, thus obtaining the first member.

The temperature of the heat treatment is appropriately adjusted according to the composition or the like of the first member, but is, for example, typically in the range of 60 to 200°C, preferably 80 to 180°C, and more preferably 90 to 160°C.

The pressure applied to the first member during the polymerization and curing is also appropriately adjusted, for example, in the range of normal pressure (atmospheric pressure) to 300 MPa, preferably 10 to 250 MPa, and more preferably 30 to 230 MPa. The temperature and pressure in the heat treatment may be optionally varied over time.

Performing the polymerization and curing by heating under pressure promotes the polymerization by heating, which reduces the amount of unpolymerized residual monomer, and prevents the inclusion of air bubbles, thus obtaining a uniform polymer.

The means of polymerization and curing by photoirradiation may vary depending on the type of photopolymerization initiator; typically, the polymerization and curing is performed by irradiation with light at a visible light wavelength, which is harmless to the human body, although it may also be performed using an ultraviolet wavelength. The wavelength of the light is, for example, preferably in the range of 250 to 700 nm, and more preferably 300 to 500 nm.

Light from an LED lamp, a halogen lamp, a xenon lamp, a metal halide lamp, a laser, a fluorescent lamp, or sunlight, for example, can be used as the light source in the above-mentioned wavelength ranges.

The irradiation time for polymerizing the polymerizable monomer-containing composition by irradiation with the above-mentioned light varies depending on the thickness, transparency, and color tone of the first member to be produced, and intensity of irradiating light; however, in general, the irradiation time may be appropriately determined according to the desired polymerization time. Preferably, the photoirradiation is performed for about 10 seconds to 10 minutes, and more preferably 1 to 6 minutes.

An example of the method for producing the first member by polymerizing and curing the polymerizable monomer-containing composition by photoirradiation is to fill a transparent silicone resin or the like with the glass fiber woven fabric and polymerizable monomer-containing composition, and then perform photopolymerization by irradiating with light at a wavelength of 300 to 500 nm from both sides for several minutes. Furthermore, in order to increase the polymerization degree, after the photo irradiation, heat treatment is preferably performed at a temperature in the range of 90 to 160°C to form a molded product.

When two or more layers of the glass fiber woven fabric are laminated, polymerization and curing of the polymerizable monomer-containing composition can be performed for these layers either one by one or all at once.

A method for producing the first member (A) comprising the first curable resin (C) and the glass fiber (D) preferably comprises the steps of:

(1) placing a glass fiber in a cavity portion of a mold;
(2) pouring a polymerizable monomer-containing composition into the cavity portion of the mold in which the glass fiber is placed, and impregnating and vacuum degassing the polymerizable monomer-containing composition; and
(3) polymerizing and curing the polymerizable monomer-containing composition to obtain a first member comprising the curable resin and glass fiber. The resulting first member can be further formed into a first member (core material) of a desired shape or size by a cutting and/or milling step.

The first member (A) has a three-point bending strength of typically 500 MPa or more, preferably 600 MPa or more, and more preferably 700 MPa or more. Setting the three-point bending strength in the above-mentioned ranges can prevent a dental prosthesis produced from the composite material for dental milling including the first member (A) from being fractured by occlusal pressure during clinical use. The lower limit is preferably high, when considering the use as a bridge. The upper limit of the three-point bending strength is not specifically limited, and may be, for example, 2500 MPa or less, preferably 2000 MPa, and more preferably 1500 MPa or less. More specifically, the three-point bending strength of the first member (A) is typically 500 MPa or more and 2500 MPa or less, preferably 600 MPa or more and 2000 MPa, and more preferably 700 MPa or more and 1500 MPa or less.

The three-point bending strength may be measured in accordance with JIS T 6526:2018 "Dental ceramic materials", and the method of measuring the three-point bending strength is described in detail in the Examples below.

The first member (A) is not limited to specific shapes and cross-sectional shapes, and the cross section of the first member is, for example, square, substantially square, rectangular, substantially rectangular, trapezoidal, substantially trapezoidal, circular, or substantially circular. The shape of the first member (core material) is not limited as long as it is three-dimensional with the above-mentioned cross section, and examples include a rectangular prism (such as a cube or cuboid) and a cylinder.

<u>(B) Second Member</u>

The second member (B) is in contact with at least a portion of the first member (A). The second member (B) can also be referred to, for example, as coating material (B1). The second member (B) can also be referred to as "second member", "(B) second member", "coating material", "(B1) coating material", or "coating material (B1)".

The second member (B) is only required to be in contact with at least a portion of the first member (A). The at least a portion of the first member (A) includes a portion of or all of the first member (A).

The second member (B) is coated or laminated on at least a portion of the first member (A).

For example, when the first member (A) is a cuboid, and when the second member (B) is in contact with the entire upper surface of the first member (A), a composite material for dental milling in the shape of a two-layer laminate (two-layer type) as shown in FIG. 1, for example, can be formed.

Alternatively, when the first member (A) has a two-layer structure in which two cuboids are layered, and when the second member (B) is in contact with the entire upper surface of the upper cuboid of the first member (A) of the two-layer structure, a composite material for dental milling in the shape of a three-layer laminate (three-layer type) as shown in FIG. 2, for example, can be formed.

Still alternatively, when the first member (A) is a cuboid, and when the second member (B) is in contact with the entire side surfaces of the first member (A), a composite material for dental milling of a shape such that the coating material (B1) coats the entire four side surfaces of the core material (A1) (coated core material type), as shown in FIG. 3, for example, can be formed. Since the size of dental prosthesis varies from individual to individual, the second member (B) (coating material) in FIG. 3 may be thicker to allow milling of a larger dental prosthesis.

The second member (B) comprises (E) a second curable resin and an inorganic filler (F). The second member (B) is preferably free from a glass fiber.

The second member (B) may be, for example, a product obtained by polymerizing and curing a second member composition comprising (E1) a (meth)acrylate-based polymerizable monomer and (F) an inorganic filler.

As described below, in the second member (B), the (meth)acrylate-based polymerizable monomer (E1) contained in the second member composition is polymerized into a curable resin by polymerizing and curing the second member composition comprising the (meth)acrylate-based polymerizable monomer (E1) and inorganic filler (F). Furthermore, since the second member composition contains the inorganic filler (F), the second member (coating material), which is a cured product of the second member composition, contains the inorganic filler (F) with the second curable resin (E).

(E1) (Meth)Acrylate-Based Polymerizable Monomer

The second member composition needs to contain the (meth)acrylate-based polymerizable monomer (E1) (hereinafter, sometimes also referred to as "polymerizable monomer" or "monomer"). The term (meth)acrylate refers to acrylate or methacrylate.

Any (meth)acrylate-based polymerizable monomers that can be used for dental applications can be used without limitation as the polymerizable monomer. Examples include monofunctional (meth)acrylates such as (meth)acrylic acid and (meth)acrylic acid esters (for example, in the case of alkyl esters, the number of carbon atoms in the alkyl groups is from 1 to 12; and in the case of esters containing aromatic groups, the number of carbon atoms in the esters containing aromatic groups is from 6 to 12. When these groups contain substituents such as a polyethylene glycol chain, the number of carbon atoms in these substituents is also included.); and polyfunctional (meth)acrylates, including di(meth)acrylates such as polyalkylene glycol di(meth)acrylates (the number of carbon atoms in the alkylene groups is from 2 to 20), ethylene glycol oligomer di(meth)acrylates (2- to 10-mers), di(meth)acrylates containing bisphenol A, and a urethane di(meth) acrylate, which is a reaction product between 2 moles of a (meth)acrylate having a hydroxyl group and 1 mole of a diisocyanate; tri(meth)acrylates such as trimethylolpropane tri(meth)acrylate; and tetra(meth)acrylates such as pentaerythritol tetra(meth)acrylate. Specifically, monomers and the like disclosed in JP-A-S50-042696 or JP-A-S56-152408 are preferable.

Examples of the monofunctional (meth)acrylates include methyl (meth)acrylate, ethyl (meth)acrylate, propyl (meth)acrylate, n-butyl (meth)acrylate, isobutyl (meth)acrylate, 2-ethylhexyl (meth)acrylate, lauryl (meth)acrylate, tridecyl (meth)acrylate, stearyl (meth)acrylate, cyclohexyl (meth)acrylate, benzyl (meth)acrylate, 2-hydroxyethyl (meth)acrylate, 2-hydroxypropyl (meth)acrylate, glycidyl (meth)acrylate, tetrahydrofurfuryl (meth)acrylate, allyl (meth)acrylate, 2-ethoxyethyl (meth)acrylate, methoxydiethylene glycol (meth)acrylate, methoxytetraethylene glycol (meth)acrylate, methoxypolyethylene glycol (meth)acrylate, phenoxydiethylene glycol (meth)acrylate, phenoxyhexaethylene glycol (meth) acrylate, glycerol (meth)acrylate, tetrahydrofurfuryl (meth)acrylate, dicyclopentenyl (meth)acrylate, isobornyl (meth) acrylate, phenyl (meth)acrylate, pentaerythritol mono(meth)acrylate, dipentaerythritol mono(meth)acrylate, caprolactone-modified tetrahydrofurfuryl (meth)acrylate, caprolactone-modified dipentaerythritol (meth)acrylate, and caprolactone-modified 2-hydroxyethyl (meth)acrylate.

Examples of the polyfunctional (meth)acrylates include di(meth)acrylates such as ethylene glycol di(meth) acrylate, diethylene glycol di(meth)acrylate, triethylene glycol di(meth)acrylate, tetraethylene glycol di(meth)acrylate, polyethylene glycol di(meth)acrylate, 1,3-butylene glycol di(meth)acrylate, 1,6-hexanediol di(meth)acrylate, neopentyl glycol di(meth)acrylate, tripropylene glycol di(meth)acrylate, polypropylene glycol di(meth)acrylate, glycerol di(meth) acrylate, bisphenol A di(meth)acrylate, ethylene oxide-modified bisphenol A di(meth)acrylate, ethylene oxide-modified bisphenol A glycidyl di(meth)acrylate, 2,2-bis(4-methacryloxypropoxyphenyl)propane, 7,7,9-trimethyl-4,13-dioxa-3,14-dioxo-5,12-diazahexadecane-1,16-diol di(meth)acrylate, neopentyl glycol hydroxypivalic acid ester di(meth)acrylate, caprolactone-modified hydroxypivalic acid neopentyl glycol ester di(meth)acrylate, trimethylolethane di(meth)acrylate, trimethylolpropane di(meth)acrylate, urethane di(meth)acrylate (1,6-bis((meth)acryloyloxy-2-ethoxycarbonylamino)-2,4,4-trimethylhexane), the reaction product of 3-chloro-2-hydroxypropyl (meth)acrylate and methylcyclohexane diisocyanate, the reaction product of 2-hydroxypropyl (meth)acrylate and methylcyclohexane diisocyanate, the reaction product of 2-hydroxypropyl (meth)acrylate and methylene bis(4-cyclohexylisocyanate), the reaction product of 2-hydroxypropyl (meth)acrylate and trimethyl hexamethylene diisocyanate, the reaction product of 2-hydroxyethyl (meth)acrylate and isophorone diisocyanate, and the reaction product of 3-chloro-2-hydroxypropyl (meth)acrylate and isophorone diisocyanate; tri(meth)acrylates such as trimethylolmethane tri(meth)acrylate, trimethylolethane tri(meth)acrylate, trimethylolpropane tri(meth)acrylate, pentaerythritol tri(meth)acrylate, and dipentaerythritol tri(meth)acrylate; and tetra(meth)acrylates such as pentaerythritol tetra(meth)acrylate and dipentaerythritol tetra(meth)acrylate.

Preferred as the (meth)acrylate-based polymerizable monomer are polyfunctional (meth)acrylates; more preferred are ethylene glycol dimethacrylate, diethylene glycol dimethacrylate (DEGDMA), triethylene glycol dimethacrylate (TEGDMA), trimethylolpropane di(meth)acrylate, urethane dimethacrylate (UDMA), and bisphenol A glycidyl dimethacrylate (Bis-GMA); and particularly preferred are DEGDMA, TEGDMA, UDMA, and Bis-GMA.

These (meth)acrylate-based polymerizable monomers can be used alone, but are preferably used as a mixture of two or more, more preferably as a mixture of two or more polyfunctional (meth)acrylates, and particularly preferably as a mixture of two or more di(meth)acrylates to adjust the viscosity. Furthermore, the same monomers as the polyfunctional (meth)acrylate-based polymerizable monomer (C1) or the low-viscosity polymerizable monomer (C2) for the first member (A) described above may be used as the (meth)acrylate-based polymerizable monomer.

The content of the polymerizable monomer contained in the second member composition is preferably 18 to 30% by mass, and more preferably 20 to 28% by mass.

In the second member composition, the (meth)acrylate-based polymerizable monomer may be mixed with other polymerizable monomers besides the above-described (meth)acrylate-based polymerizable monomer and polymerized, for ease of polymerization, regulation of viscosity, or regulation of other physical properties. The low-viscosity polymerizable monomers (C2) for the first member described above may also be used as other polymerizable monomers besides the (meth)acrylate-based polymerizable monomer. That is, the second member (B) (coating material) may be obtained by polymerizing and curing a second member composition comprising a second polymerizable monomer-containing composition comprising the (meth)acrylate-based polymerizable monomer (E1); and the inorganic filler (F).

The same monomers as the polyfunctional (meth)acrylate-based polymerizable monomer (C1) and the low-viscosity polymerizable monomer (C2) used for the first member may be used as the (meth)acrylate-based polymerizable monomer (E1).

### (F) Inorganic Filler

The inorganic filler (F) is not specifically limited as long as it is a commonly used inorganic filler, and examples include kaolin, talc, quartz, silica (for example, colloidal silica), alumina, aluminosilicate glass, fluoroaluminosilicate glass, silicon nitride, barium sulfate, calcium phosphate, glass powder, zirconia, and zirconium silicate. In addition to these, composite metal oxide fillers, inorganic fillers containing fluorides, and ultrafine $SiO_2$ fillers having an average particle diameter of 0.1 $\mu$m or less can also be used.

The second member (B) preferably contains at least one inorganic filler (F) selected from the group consisting of (f1) a composite metal oxide filler; (f2) an inorganic filler containing a fluoride; and (f3) an ultrafine $SiO_2$ filler having an average particle diameter of 0.1 $\mu$m or less, and more preferably contains all of (f1) a composite metal oxide filler; (f2) an inorganic filler containing a fluoride, and (f3) an ultrafine $SiO_2$ filler having an average particle diameter of 0.1 $\mu$m or less.

The inorganic filler (F) is not limited to specific compositions, shapes, particle size distributions, average particle diameters, and the like. The inorganic filler (F) has an average particle diameter of preferably 50 $\mu$m or less, more preferably 10 $\mu$m or less, and still more preferably 1 $\mu$m or less. The inorganic filler (F) is free from a glass fiber.

The inorganic filler (F) may be contained in the second member composition at a proportion of 50 to 90% by mass, preferably 60 to 85% by mass, and more preferably 70 to 80%.

### (f1) Composite Metal Oxide Filler

The composite metal oxide filler (f1) is a composite metal oxide filler containing $SiO_2$, $ZrO_2$, and $Al_2O_3$ (hereinafter, sometimes also referred to as "composite metal oxide filler"). The composite metal oxide filler (f1) is preferably a filler formed of secondary particles having an average particle diameter of 2 to 8 $\mu$m obtained by sintering and partially bonding primary particles having an average particle diameter of 0.1 to 0.9 $\mu$m.

The content of the composite metal oxide filler (f1) in the second member composition is 21 to 61% by mass, preferably 25 to 55% by mass, and more preferably 30 to 50% by mass.

The composite metal oxide filler (f1) may be only a single composite metal oxide filler, or a mixture of two or more different composite metal oxide fillers.

The composite metal oxide filler (f1) is characterized by having a refractive index (nD) equal to or similar to the refractive index (nD) of the resin forming a matrix of the second member composition. The composition ratio of $SiO_2$, $ZrO_2$, and $Al_2O_3$ can be adjusted so that the difference between the refractive indices (nD) of the composite metal oxide filler (f1) and the resin falls within the range of $\pm$ 0.006.

The refractive index (nD) of the composite metal oxide filler (f1) varies depending on the refractive index of the resin to be used, but can be controlled within the range of 1.49 to 1.52 in the case of (meth)acrylate-based polymerizable monomers commonly used in dentistry. These properties of the filler can impart high transparency to the second member composition.

The composite metal oxide filler (f1), i.e., the agglomerated secondary particles in which the sintered primary particles are bonded, is produced by the steps of finely pulverizing particles composed of a porous amorphous $SiO_2$-$ZrO_2$-$Al_2O_3$ material prepared by a sol-gel process to the above-mentioned particle diameter to obtain fine gel particles; agglomerating the fine gel particles; and firing the agglomerates.

The primary particles are sufficiently sintered by the firing, but form secondary particles in which the sintered primary particles are weakly bonded, thus producing the composite metal oxide filler used in the present invention. The second member (cured product) obtained using the composite metal oxide filler (f1) is excellent in both ease of grinding and surface lubricity after grinding and/or polishing.

The second member (coating material) has a large bending strength because it contains the filler (secondary particles, average particle diameter = 2 to 8 $\mu$m) in which primary particles having an average particle diameter of about 0.1 to 0.9 $\mu$m are bonded. The high mechanical strength is believed to be because the filler (secondary particles) has surface irregularities, and the polymerizable monomer enters the surface irregularities and is then cured to produce a fitting effect (also referred to as an anchor effect).

In the filler used in the present invention, the surface of the primary particles is sufficiently sintered because the $SiO_2$-$ZrO_2$-$Al_2O_3$-based fine gel particles (primary particles after sintering) are dried and then sintered at a high temperature. Thus, the filler has a small specific surface area, so that the second member (cured product) obtained using the filler has a low water absorption rate, and has excellent durability under wet conditions such as in the oral cavity.

The composite metal oxide filler (f1) used in the present invention is produced by a method comprising the steps of co-precipitating and drying a mixture of an alkoxysilane, a hydrolyzable zirconium compound, and a hydrolyzable aluminum compound by a sol-gel process to obtain a gel (step A1); pulverizing the gel into fine particles (primary particles) (step A2); and forming secondary particles by firing the primary particles (step A3), the method optionally further comprising the step of surface-treating the secondary particles with a silane coupling agent (step A4).

Specifically, in step A1, the alkoxysilane, hydrolyzable aluminum compound, and hydrolyzable zirconium compound are uniformly mixed in a solvent to prepare a solution containing 50 to 95% by mass (preferably 60 to 85% by mass) of $SiO_2$, 0.1 to 30% by mass (preferably 10 to 20% by mass) of $ZrO_2$, and 0.1 to 30% by mass (preferably 0.3 to 10% by mass) of $Al_2O_3$, and the solution is mixed with an alkaline solution to simultaneously hydrolyze each component to precipitate gel particles of the reaction product.

In the composition of the composite metal oxide filler (f1), the refractive index (nD) of $SiO_2$ is 1.46, the refractive index (nD) of $ZrO_2$ is 2.2, and the refractive index (nD) of $Al_2O_3$ is 1.76. Depending on the content of each component, the refractive index (nD) of the composite metal oxide filler (f1) changes substantially according to the additive rule. To increase the transparency of the second member (coating material), the refractive index needs to be similar to the refractive index of the resin (1.48 to 1.52), and it is important to select the content of each component to be an appropriate value. The $ZrO_2$ component can also be added to impart radio-opacity to the dental material.

Components such as $TiO_2$, $CeO_2$, and $Y_2O_3$ that increase the refractive index (nD) of the composite metal oxide filler (f1) can also be introduced, but preferably in a small amount, i.e., 3% by mass or less, based on the final total weight of the filler.

Examples of the alkoxysilane include, but are not specifically limited to, tetraalkoxysilane compounds represented by the general formula: $Si(OR)_4$ (wherein R represents an alkyl group, preferably methyl, ethyl, n-propyl, isopropyl, n-butyl, tert-butyl, or the like); and partially hydrolyzed oligomers of tetraalkoxysilanes. Among these, the alkoxysilane is preferably ethoxysilane, methoxysilane, or a methyl silicate oligomer ($SiO_2$ content = 52 wt%, $(CH_3O)_{10}Si_4$ = approximately tetramer, MS51 manufactured by Mitsubishi Chemical Co., Ltd.), and is more preferably a methyl silicate oligomer in that it is inexpensive and easy to handle.

Examples of the hydrolyzable aluminum compound include, but are not specifically limited to, aluminum salts such as aluminum nitrate ($Al(NO_3)_3$), which is inexpensive and easily thermally decomposable, aluminum acetate ($Al(OAc)_3$), and aluminum acetylacetonate; and trialkoxyaluminum compounds represented by $Al(OR)_3$ (wherein R represents an alkyl group, preferably n-propyl, isopropyl, n-butyl, tert-butyl, or the like). These aluminum salts can be typically used as an aqueous solution.

Examples of the hydrolyzable zirconium compound include tetrazirconium compounds represented by the general formula: $Zr(OR')_4$ (wherein R' represents an alkyl group, preferably methyl, ethyl, n-propyl, isopropyl, n-butyl, tert-butyl, or the like); $ZrO(NO_3)_2 \cdot nH_2O$; and $ZrOCl_2 \cdot nH_2O$. Among these, preferred is (are) $ZrO(NO_3)_2 \cdot nH_2O$ or $ZrOCl_2 \cdot nH_2O$ and $ZrO(NO_3)_2 \cdot nH_2O$, wherein n represents an integer of 1 to 10.

A specific method for producing the composite metal oxide filler is as follows. Aluminum nitrate nonahydrate ($Al(NO_3)_3 \cdot 9H_2O$) is dissolved in water or an alcohol, and a zirconium compound (for example, an aqueous $ZrO(NO_3)_2$ solution) is added to the solution and sufficiently mixed. An alkoxysilane, for example, a methyl silicate oligomer, is added to the mixture to obtain a uniform and transparent raw material mixture solution. The inorganic oxide content in the resulting raw material mixture solution is in the range of about 1 to 35% by mass, and preferably in the range of about 3 to 10% by mass. This is because, if the content of water or the organic solvent is high, drying requires time and is uneconomical, and if the content of the solvent is low, the subsequent neutralization and stirring operations will be difficult.

The raw material mixture solution (sol) prepared as described above can be gelled by hydrolysis and a co-precipitation reaction by adding an alkaline solution.

The alkaline solution is not specifically limited, but is preferably ammonia water, for example, in that it dissolves the raw material mixture solution, is dissolved in water at an arbitrary ratio, and does not remain in the filler after drying and heat treatment. The amount of ammonia water required is such that when mixed with the raw material mixture solution, the resulting mixture is basic, and in general, has a pH of about 7 to 9, and preferably a pH of about 8. For example, approximately a two-fold dilution of commercially available ammonia water (content: 35 wt%) can be used.

The method of mixing the raw material mixture solution and the alkaline solution is not specifically limited; for example, preferably, the raw material mixture solution and the alkaline solution are added all at once, in order to prevent nonuniformity of the components in the coprecipitate, which can be caused by different hydrolysis conditions with the alkali for each raw material component. The stirring speed, reaction temperature, and reaction time are also not specifically limited; when vigorous mixing is performed for the purpose of a uniform reaction, and rapid neutralization is achieved to obtain an aggregate of co-precipitated fine particles (jelly-like gel), the nonuniformity of the components can be prevented.

The sol-gel obtained by the above-described procedure is placed in a common evaporator or oven to evaporate and remove the solvent, excess ammonia, water, and the like, and to be dried. The drying temperature is not specifically limited and is, for example, in the range of 40 to 150°C, and preferably 70 to 120°C.

Next, the dried gel is washed with water to remove by-products such as ammonium nitrate. For example, the gel is sufficiently dried in a convection oven or the like; then, ethanol is added to the dried product and the mixture is wet-pulverized into fine gel particles having an average particle diameter of 0.1 to 0.9 $\mu$m using a planetary mill, a bead mill, or the like; and the ethanol is removed by evaporation and the resulting product is dried to obtain a fine gel powder. The fine powder is pulverized into particles having an average particle diameter of 3 to 20 $\mu$m using an airflow pulverizer such as a jet mill, and the resulting particles are fired in an electric furnace. In Patent Literature 4, the particles are produced by pulverizing the fine powder by collision with alumina balls in a planetary mill before firing. However, when using a planetary mill, the fine powder is vigorously compacted by the alumina balls, which makes the inorganic filler after firing hard. Thus, an abrasive material containing diamond grains having high abrasive force needs to be used to obtain a sufficient gloss during final polishing of the composite material for dental milling (cured product). On the other hand, the airflow pulverizer used in the present invention performs pulverization by collision between powders at high speed, without using a medium such as alumina balls. Thus, the particles do not become excessively hard, so that the composite material for dental milling is characterized by being easily polished, and achieving a sufficient gloss by using a general-purpose abrasive material containing alumina grains having low abrasive force.

In the firing of the particles, the sintering of primary particles and the formation of secondary particles (average particle diameter: 3 to 10 $\mu$m) in which the primary particles are bonded are important. The optimal heat treatment conditions (temperature, time, and the like) are appropriately selected according to the contents of the $SiO_2$, $ZrO_2$, and $Al_2O$ components.

For example, the heating rate is at most about 20°C per minute, and is typically preferably about 3 to 10°C per minute. The firing temperature is about 800 to 1200°C, preferably about 1000 to 1190°C, and more preferably 1050 to 1150°C.

The secondary particles produced by the above-described method are adjusted to have an appropriate particle size distribution by a method such as crushing or blending. The inorganic filler of the secondary particles is in the form of irregular particles (particle diameter = 1 to 50 $\mu$m) in which the sintered primary particles having an average particle diameter of about 0.1 to 0.9 $\mu$m are bonded together by neck formation. The secondary particles are free of sharp edges, have a broad particle size distribution and are nonuniform in particle size, and have surface irregularities. The secondary particles after firing can be optionally crushed using the airflow pulverizer, and regulated to an average particle diameter of 2 to 8 $\mu$m.

The average particle diameter and particle size distribution can be measured using, for example, a laser diffraction particle size distribution analyzer (SALD-2200, manufactured by Shimadzu Corporation). The average particle diameter refers to the volume average particle diameter because the particle size distribution determined by the laser diffraction/scattering method is measured on a volume basis.

The particle sizes of the fine gel particles (primary particles) before firing and the particles (secondary particles) before firing are measured by placing the powders into a particle size distribution analyzer, using distilled water as the solvent and at a refractive index of 1.45 $\pm$ 0.10, and subjecting the powders to ultrasonic dispersion for 5 minutes, followed by measurement. The particle size of the particles (secondary particles) after firing is measured at a refractive index of 1.50 $\pm$ 0.10.

(f2) Inorganic Filler Containing Fluoride

The inorganic filler containing a fluoride (f2) is an inorganic filler containing a fluoride and having an average particle diameter of 1 $\mu$m or less (hereinafter, sometimes also referred to as "fluorine filler").

The fluorine filler (f2) is not specifically limited as long as it is a compound that releases fluorine ions in an aqueous solution, and may be, for example, any known inorganic filler containing a fluoride that has an average particle diameter of 0.1 to 0.9 $\mu$m. Specifically, examples of the fluorine filler (f2) include alkali metal fluorides such as sodium fluoride, potassium fluoride, and lithium fluoride; alkaline earth metal fluorides such as calcium fluoride and strontium fluoride; silicon fluoride compounds such as fluorosilicates; zinc fluoride compounds; fluorinated phosphate compounds such as sodium monofluorophosphate, lithium monofluorophosphate, ammonium monofluorophosphate, and aluminum monofluorophosphate; and composite compounds thereof. Among these, the fluorine filler (f2) preferably contains 5 to 50% by mass of fluorine (F), based on the fluorine filler (a sum of 5 to 30% by mass of CaO, 5 to 30% by mass of SrO, 10 to 70% by mass of $SiO_2$, 10 to 50% by mass of $Al_2O_3$, 0 to 20% by mass of ZnO, 0 to 10% by mass of $Na_2O$, and 0 to 10% by mass of $P_2O_5$). The fluorine filler (f2) may have an average particle diameter of, for example, 2 $\mu$m or less, and preferably in the range of 0.1 to 1 $\mu$m, although not specifically limited thereto.

The fluorine filler (f2) may be only a single fluorine filler, or a mixture of two or more different fluorine fillers.

The content of the fluorine filler (f2) in the second member (coating material) is preferably 7 to 50% by mass, more preferably 10 to 45% by mass, and still more preferably 12 to 40% by mass.

(f3) Ultrafine $SiO_2$ Filler Having an Average Particle Diameter of 0.1 $\mu$m or Less

The ultrafine $SiO_2$ filler having an average particle diameter of 0.1 $\mu$m or less (hereinafter, sometimes also referred to as "ultrafine $SiO_2$ filler") is not specifically limited as long as it is a known $SiO_2$ filler having an average primary particle diameter of 0.1 $\mu$m or less, and examples include colloidal silica and fumed silica. The average particle diameter of the ultrafine $SiO_2$ filler may be 0.1 $\mu$m or less, and is preferably in range of 0.01 to 0.1 $\mu$m.

The ultrafine $SiO_2$ filler (f3) may be only a single ultrafine $SiO_2$ filler, or a mixture of two or more different ultrafine $SiO_2$ fillers.

The content of the ultrafine $SiO_2$ filler (f3) in the second member (coating material) is preferably 1 to 10% by mass, and more preferably 2 to 8% by mass.

The ultrafine $SiO_2$ filler (f3) is a $SiO_2$ filler, which does not contain $ZrO_2$ and $Al_2O_3$, whereas the inorganic filler containing $SiO_2$, $ZrO_2$, and $Al_2O_3$ as the component A is composed of a composite metal oxide necessarily containing each of $SiO_2$, $ZrO_2$, and $Al_2O_3$.

The fluorine filler (f2) is an inorganic filler necessarily containing a fluoride, whereas the fillers (f1) and (f3) do not contain a fluoride, and therefore, the three types of fillers can be clearly distinguished in terms of components.

Optional Additives

The coating material composition may optionally further contain optional additives.

Examples of additives include a polymerization initiator, a polymerization accelerator, a coloring pigment, an opacifying agent, a fluorescent material, an opalizing agent, a polymerization inhibitor, an antioxidant, an antibacterial agent, an X-ray contrast agent, a stabilizer, an ultraviolet absorber, a discoloration inhibitor, and various other known additives. These additives can be used alone or in appropriate combinations of two or more.

The coating material composition preferably contains a polymerization initiator.

The polymerization initiator is not specifically limited as long as it is a commonly used polymerization initiator, and is particularly preferably a polymerization initiator used for dental applications. In general, different types of polymerization initiators are used depending on the polymerization means for the polymerizable monomer. A photopolymerization initiator, a thermal polymerization initiator, and the like may be used as the polymerization means.

The photopolymerization initiator is not specifically limited, and may be a photopolymerization initiator that reacts with ultraviolet or visible light to generate radicals. Specific examples include $\alpha$-diketones such as diacetyl, acetylbenzoyl, benzyl, camphorquinone (CQ), 9,10-phenanthrenequinone, and acenaphthenequinone; benzoin alkyl ethers such as benzoin methyl ether, benzoin ethyl ether, and benzoin propyl ether; thioxanthone compounds such as 2,4-diethylthioxanthone, 2,4,6-trimethylbenzoyl diphenylphosphine oxide, and methylthioxanthone; and benzophenone-based compounds such as benzophenone, p,p'-dimethylaminobenzophenone, and p,p'-dimethoxyaminobenzophenone.

The thermal polymerization initiator is not specifically limited, and may be a known thermal polymerization initiator, such as a peroxide or an azo compound. Specific examples include benzoyl peroxide (BPO), ketone peroxides, peroxyketals, hydroperoxides, dialkyl peroxides, diacyl peroxides, peroxyesters, peroxydicarbonates, 2,2'-azobisisobutyronitrile, 2,2'-azobis-2,4-dimethylvaleronitrile, 4,4'-azobis-4-cyanovaleric acid, 1,1'-azobis-1-cyclohexanecarbonitrile, dimethyl-2,2'-azobisisobutyrate, and 2,2'-azobis-(2-aminopropane) dihydrochloride.

In the polymerization of the (meth)acrylate-based polymerizable monomer used for the second member (coating material), the polymerization initiator described above may be used in combination with a polymerization accelerator.

The polymerization accelerator is generally used in combination with a photopolymerization initiator. Examples of the polymerization accelerator include, but are not specifically limited to, 2-(dimethylamino)ethyl methacrylate (DMAEMA), ethyl 2-(dimethylamino)benzoate methacrylate (DMABE), and n-butoxyethyl 2-(dimethylamino)benzoate methacrylate.

Furthermore, the inorganic filler (F) is preferably subjected to surface treatment to improve the mechanical strength, abrasion resistance, and water resistance of the composite material for dental milling. Any known methods may be employed without limitation as the surface treatment agent and the surface treatment method.

Examples of the surface treatment agent include, but are not specifically limited to, compounds used as surface-modifying agents of inorganic oxides, for example, silane coupling agents, such as 3-(meth)acryloyloxypropyltrimethoxysilane, 4-(meth)acryloyloxybutyltrimethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 12-(meth)acryloyloxydodecyltrimethoxysilane, 13-(meth)acryloyloxytridecyltrimethoxysilane, 14-(meth)acryloyloxytetradecyltrimethoxysilane, vinyltrimethoxysilane, vinyltriethoxysilane, 3-aminopropylethoxysilane, 3-chloropropyltrimethoxysilane silyl isocyanate, and vinyltrichlorosilane. 3-Methacryloyloxypropyltrimethoxysilane ($\gamma$-MPTS) is preferred, for example.

Examples of methods of treating the inorganic filler (F) with a surface treatment agent include, but are not specifically limited to, a method in which each filler and the surface treatment agent are heated to reflux in a solvent such as an alcohol for several tens of minutes to about 10 hours, and preferably in the range of 1 to 5 hours. If it is necessary to accelerate the hydrolysis of the surface treatment agent, a method may be employed in which water or acidic water such as acetic acid is added to the solvent, and the mixture is heated to reflux for a time in the above-mentioned range, and then the solvent is removed and the resulting product is dried under normal pressure or under reduced pressure.

The amount of the surface treatment agent is in the range of 0.1 to 50 parts by mass, and preferably 1 to 30 parts by mass, per 100 parts by mass of each of the fillers. Each of the fillers after the surface treatment shows almost no change in particle diameter or particle size distribution as compared to the filler before the treatment.

In addition to the components (E) and (F), the second member (B) may contain the additives described above as long as they do not interfere with the effects of the present invention.

The coloring pigment (and the opacifying agent) may be any known materials for common dental treatment applications, in order to mimic natural teeth. Examples include iron oxide-based coloring pigments, organic pigments, zirconium oxide pigments, titanium white, and titanium yellow. Specific examples include iron oxide, zirconium oxide, and titanium oxide.

The second member (B) may be not only a single type of second member, but also a combination of two or more types of second members. When the second member (B) includes two types of second members, it may be composed of, for example, a second member (B-1) and a second member (B-2). Alternatively, when the second member (B) includes three or more types of second members, it may be composed of, for example, the second member (B-1), the second member (B-2), a second member (B-3), and the like. The second members (B-1), (B-2), (B-3), and the like may be different in terms of, for example, the type of coloring pigment contained and the content thereof. The second member (B) may be obtained by laminating a plurality of second members having different color tones to form a gradation structure.

Method for Producing Second Member Composition

The second member composition, which comprises (E1) a (meth)acrylate-based polymerizable monomer and (F) an inorganic filler, can be produced by mixing 10 to 50% by mass of the (meth)acrylate-based polymerizable monomer (E1) and 50 to 90% by mass of the inorganic filler (F).

The content of the inorganic filler (F) is typically 100 to 900 parts by mass, preferably 120 to 700 parts by mass, and more preferably 150 to 500 parts by mass, per 100 parts by mass of the (meth)acrylate-based polymerizable monomer (E1).

The proportions (mixing ratio) of the components in the second member composition can be appropriately adjusted according to the viscosity and the purpose of use. For example, when the second member composition contains the inorganic fillers (f1) to (f3), it may contain 42 to 600 parts by mass (preferably 50 to 250 parts by mass) of the composite metal oxide filler (f1), 14 to 500 parts by mass (preferably 20 to 450 parts by mass) of the fluorine filler (f2), and 2 to 100 parts by mass (preferably 4 to 80 parts by mass ) of the ultrafine $SiO_2$ filler (f3), per 100 parts by mass of the (meth)acrylate-based polymerizable monomer, and may optionally further contain additives such as a polymerization initiator, a polymerization accelerator, a coloring pigment, an opacifying agent, an opalizing agent, a fluorescent material, a polymerization inhibitor, an antioxidant, an antibacterial agent, an X-ray contrast agent, a stabilizer, an ultraviolet absorber, and a discoloration inhibitor, as appropriate. When the dental composition contains a polymerization initiator, it needs to be handled with care, and must be stored in an airtight, dark, and low-temperature storage environment.

A method for producing the second member composition may include the steps of placing a predetermined amount of each of the components in a container, and sufficiently kneading and dispersing the mixture to give a paste; and kneading the paste under reduced pressure or stirring the paste under vacuum. This gives a uniform and air bubble-free, clay-like or paste-like dental composition.

The second member composition is polymerized according to known polymerization methods (through light and heat) to obtain a cured product (second member).

The second member (B) has a three-point bending strength that is lower than that of the first member (A), typically less than 500 MPa. Preferably, the three-point bending strength of the second member (B) is also high, when considering the bending strength of the dental prosthesis produced from the composite material for dental milling including the second member (B). The upper limit of the three-point bending strength of the second member (B) is typically less than 500 MPa, and may be 400 MPa or less, 300 MPa or less, 250 MPa or less, or 200 MPa or less. The lower limit of the three-point bending strength is, for example, 100 MPa or more, preferably 120 MPa or more, and more preferably 180 MPa or more.

More specifically, the three-point bending strength of the second member (B) is typically 100 MPa or more and less than 500 MPa, preferably 110 MPa or more and 400 MPa or less, and more preferably 120 MPa or more and 250 MPa or less.

The three-point bending strength may be measured in accordance with JIS T 6526:2018 "Dental ceramic materials", and the method of measuring the three-point bending strength is described in detail in the Examples below.

Production of Composite Material for Dental Milling

For example, the first member (A) is cut into a bar or plate shape, and a surface treatment agent containing a silane coupling agent is applied to a portion of the first member (A) to be brought in contact with the second member (B) and dried. Then, the first member (A) surface-treated and dried is placed in the center or bottom portion of the cavity portion (16 mm in height × 19 mm in width × 100 mm in length) of a mold, and the cavity portion of the mold is filled with the second member composition, which is then subjected to polymerization by heating. In this manner, a composite material for dental milling can be obtained as an integral piece of the first member (A) (core material) and the second member (B) (coating material). The resulting composite material for dental milling can be further formed into a material of a desired shape or size by a cutting and/or milling step.

The composite material for dental milling is of a shape (type) such as a laminate or a coated core material type, for example.

Examples of shapes of the composite material for dental milling include:

a shape in which at least a portion of the surface of the first member (core material) is in contact with the second member (coating material);

a shape in which the entire upper surface of the first member that is a cuboid is in contact with the second member (for example, a two-layer laminate as shown in FIG.1);

a shape in which the first member that is a cuboid has a two-layer structure, and the entire upper surface of the upper cuboid of the first member is in contact with the second member (for example, a three-layer laminate as shown in FIG. 2); and

a shape in which the entire four side surfaces of the first member (core material) that is a cuboid are in contact with the second member (coating material) (for example, the shape as shown in FIG. 3 (coated core material type)).

When the composite material for dental milling is of the coated core material type, if the first member (core material) has a small thickness (height), it needs to be increased in width to ensure a certain cross-sectional area. In this case, it is difficult to mill the composite material for dental milling so that the first member (core material) enters the width of the connecting portion of a dental bridge. Therefore, the first member (core material) preferably has an appropriate thickness.

The first member (A) (core material) in the composite material for dental milling preferably has a cross-sectional area of typically 3 to 330 $mm^2$, preferably 3.1 to 310 $mm^2$, and more preferably 3.2 to 300 $mm^2$, in view of facilitating the production of a dental prosthesis by milling. The height and width of the first member (A) (core material) in the composite material for dental milling are not specifically limited as long as the cross-sectional area falls in the above-mentioned ranges. The first member (A) (core material) has a height of typically 0.5 to 16.5 mm, preferably 0.7 to 16 mm, and more preferably 1 to 15.5 mm. The first member (A) (core material) has a width of typically 0.5 to 20 mm, preferably 0.7 to 19.5 mm, and more preferably 1 to 19 mm. Setting the cross-sectional area of the first member (A) (core material) in the composite material for dental milling within the above-mentioned ranges imparts a three-point bending strength required for a dental bridge to the dental prosthesis produced from the composite material for dental milling.

The height and width of the second member (B) in the composite material for dental milling are not specifically limited. For example, the second member (B) has a height of typically 0.1 to 30 mm, preferably 0.5 to 25 mm, and more preferably 1 to 20 mm. The second member (B) (coating material) has a width of typically 0.1 to 30 mm, preferably 0.5 to 25 mm, and more preferably 1 to 20 mm.

The first member (A) in the composite material for dental milling has a volume of typically 100 to 20000 $mm^3$,

preferably 120 to 18000 mm$^3$, and more preferably 140 to 17000 mm$^3$. The first member (A) in the composite material for dental milling is not limited to specific lengths, and may, for example, have a length that can be calculated from the above-mentioned range of the cross-sectional area of the first member (A) and the above-mentioned range of the volume of the first member (A).

Setting the cross-sectional area of the first member (A) in the composite material for dental milling within the above-mentioned ranges imparts a three-point bending strength required for a dental bridge to the dental prosthesis produced from the composite material for dental milling.

The second member (B) in the composite material for dental milling is not limited to specific lengths, and may have a length of, for example, 30 to 60 mm.

In the composite material for dental milling, the cross-sectional area ratio between the first member (A) (core material) and the second member (B) (coating material) is not specifically limited; for example, the ratio (cross-sectional area ratio) between the cross-sectional area of the first member (A) (core material) and the cross-sectional area of the second member (B) (coating material) is typically 1:0.05 to 100, preferably 1:0.1 to 90, and more preferably 1:0.2 to 85. That is, the ratio of the cross-sectional area of the second member (B) (coating material) to the cross-sectional area of the first member (A) (core material) is typically 0.05 to 100, preferably 0.1 to 90, and more preferably 0.2 to 85.

When the composite material for dental milling is a laminate, the cross-sectional areas, heights, widths, and volumes of the first member (A) and the second member (B) are as follows.

The cross-sectional area of the first member (A) in the composite material for dental milling is typically 2 to 330 mm$^2$, preferably 2.5 to 310 mm$^2$, and more preferably 3.5 to 300 mm$^2$.

The height of the first member (A) is typically 0.7 to 16.5 mm, preferably 0.8 to 16 mm, and more preferably 0.9 to 15.5 mm.

The width of the first member (A) is typically 3 to 20 mm, preferably 3.5 to 19.5 mm, and more preferably 4 to 19 mm.

The volume of the first member (A) is typically 80 to 20000 mm$^3$, preferably 100 to 18000 mm$^3$, and more preferably 140 to 17000 mm$^3$.

When the first member (A) has a laminated structure of two or more layers, the cross-sectional area, height, and volume of the entire first member (A) may be appropriately adjusted to fall within the above-mentioned numerical ranges.

The cross-sectional area of the second member (B) in the composite material for dental milling is typically 0.6 to 330 mm$^2$, preferably 0.7 to 310 mm$^2$, and more preferably 0.8 to 300 mm$^2$.

The height of the second member (B) is typically 0.2 to 16.5 mm, preferably 0.5 to 16 mm, and more preferably 0.8 to 15.5 mm.

The width of the second member (B) is typically 3 to 20 mm, preferably 3.5 to 19.5 mm, and more preferably 4 to 19 mm.

The volume of the second member (B) is typically 20 to 11000 mm$^3$, preferably 70 to 10000 mm$^3$, and more preferably 120 to 9000 mm$^3$.

When the composite material for dental milling is a laminate, the cross-sectional area ratio between the first member (A) and the second member (B) is not specifically limited; for example, the ratio (cross-sectional area ratio) between the cross-sectional area of the first member (A) and the cross-sectional area of the second member (B) is typically 1:0.05 to 4.5, preferably 1:0.055 to 4, and more preferably 1:0.06 to 3.5. That is, the ratio of the cross-sectional area of the second member (B) to the cross-sectional area of the first member (A) is typically 0.05 to 4.5, preferably 0.055 to 4, and more preferably 0.06 to 3.5.

When the composite material for dental milling is of the coated core material type, the core material (A1) in the composite material for dental milling preferably has a cross-sectional area of typically 3 to 14 mm$^2$, preferably 3.1 to 10 mm$^2$, and more preferably 3.2 to 8 mm$^2$, in view of facilitating the production of a dental prosthesis by milling. The height and width of the core material (A1) in the composite material for dental milling are not specifically limited as long as the cross-sectional area falls in the above-mentioned ranges. The core material (A1) has a height of typically 0.5 to 6 mm, preferably 0.7 to 5 mm, and more preferably 1 to 4.5 mm. The core material (A1) has a width of typically 0.5 to 6 mm, preferably 0.7 to 5 mm, and more preferably 1 to 4.5 mm. Setting the cross-sectional area of the core material (A1) in the composite material for dental milling within the above-mentioned ranges imparts a three-point bending strength required for a dental bridge to the dental prosthesis produced from the composite material for dental milling.

When the composite material for dental milling is of the coated core material type, the height and width of the coating material (B1) are not specifically limited. For example, the coating material (B1) has a height of typically 0.1 to 30 mm, preferably 0.5 to 25 mm, and more preferably 1 to 20 mm. The coating material (B1) has a width of typically 0.1 to 30 mm, preferably 0.5 to 25 mm, and more preferably 1 to 20 mm.

When the composite material for dental milling is of the coated core material type, the core material (A1) has a volume of typically 100 to 460 mm$^3$, preferably 120 to 430 mm$^3$, and more preferably 140 to 360 mm$^3$. The core material (A1) in the composite material for dental milling is not limited to specific lengths, and may, for example, have a length that can be calculated from the above-mentioned range of the cross-sectional area of the core material (A1) and the above-mentioned range of the volume of the core material (A1).

Setting the cross-sectional area of the core material (A1) in the composite material for dental milling within the above-mentioned ranges imparts a three-point bending strength required for a dental bridge to the dental prosthesis produced from the composite material for dental milling.

The coating material (B1) in the composite material for dental milling is not limited to specific lengths, and may have a length of, for example, 30 to 60 mm.

In the composite material for dental milling, the cross-sectional area ratio between the core material (A1) and the coating material (B1) is not specifically limited; for example, the ratio (cross-sectional area ratio) between the cross-sectional area of the core material (A1) and the cross-sectional area of the coating material (B1) is typically 1:0.05 to 100, preferably 1:0.5 to 90, and more preferably 1:1 to 85. That is, the ratio of the cross-sectional area of the coating material (B1) to the cross-sectional area of the core material (A1) is typically 0.05 to 100, preferably 0.5 to 90, and more preferably 1 to 85.

Dimensions of the composite material for dental milling include, for example, a cuboid with a height of 15.5 mm, a width of 19 mm, and a length of 40 mm to provide a three-unit bridge by milling; and a cuboid with a height of 15.5 mm, a width of 19 mm, and a length of 55 mm to provide a four-unit bridge by milling; but are not limited to these sizes.

The composite material for dental milling has a three-point bending strength of 100 MPa or more, preferably 200 MPa or more, more preferably 300 MPa or more, and particularly preferably 500 MPa or more. Setting the three-point bending strength in the above-mentioned ranges can prevent a dental prosthesis produced from the composite material for dental milling of the present invention from being fractured by occlusal pressure during clinical use. The lower limit is preferably high, when considering the use as a frame portion of a bridge. The upper limit of the three-point bending strength is not specifically limited, and may be, for example, 2000 MPa or less, preferably 1500 MPa or less, and more preferably 1000 MPa or less. More specifically, the three-point bending strength of the composite material for dental milling of the present invention is 100 MPa or more and 2000 MPa or less, preferably 200 MPa or more and 1500 MPa or less, and more preferably 500 MPa or more and 1000 MPa or less.

The three-point bending strength may be measured in accordance with JIS T 6526:2018 "Dental ceramic materials", and the method of measuring the three-point bending strength is described in detail in the Examples below.

The composite material for dental milling has a three-point bending elastic modulus of typically 14 to 33 GPa, preferably 15 to 26 GPa, and more preferably 16 to 21 GPa. When the three-point bending elastic modulus is set within the range of the three-point bending elastic modulus (about 15 GPa) of human tooth dentin and the three-point bending elastic modulus (about 84 GPa) of human tooth enamel of an abutment tooth for fixing a dental prosthesis produced from the composite material for dental milling, stress concentration is less likely to occur during occlusion, which can prevent breakage of the dental prosthesis, root fractures, and the like.

The three-point bending elastic modulus can be calculated from a stress-strain curve created by measuring the three-point bending strength by the method as defined in JIS T 6526:2018 "Dental ceramic materials", and the method of measuring the three-point bending elastic modulus is described in detail in the Examples below.

The composite material for dental milling has a density of typically 1 to 4 $g/cm^3$, preferably 1.2 to 3.5 $g/cm^3$, and more preferably 1.5 to 3 $g/cm^3$.

Since human tooth dentin has a density of about 2 $g/cm^3$, and human tooth enamel has a density of about 3 $g/cm^3$, the density of the composite material for dental milling is set in the above-mentioned ranges similar to that of the human tooth substances, so that a dental prosthesis produced from the composite material for dental milling is less likely to cause loss of occlusal balance, and therefore, can possibly reduce health hazards (such as abrasion or fracture of teeth, temporomandibular disorders, stiff shoulders, and stress) caused by loss of occlusal balance.

The density may be measured by the method as defined in ISO17304:2013, and the method of measuring the density is described in detail in the Examples below.

Production of Dental Prosthesis

The composite material for dental milling of the present invention can be used as a dental prosthesis by a dentist or a dental technician. The composite material for dental milling of the present invention may be manually milled, but may be milled using, for example, a dental CAD/CAM system (apparatus), which allows a dental prosthesis with a crown shape to be produced by a single milling operation.

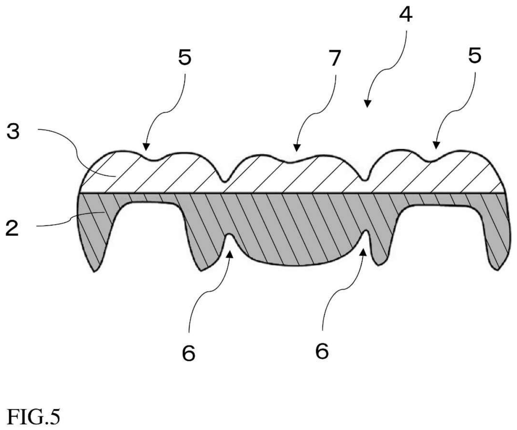

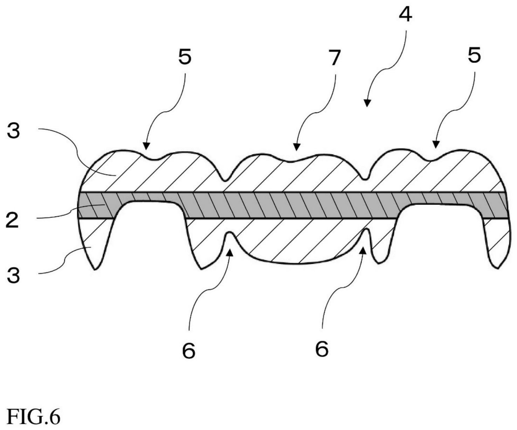

Examples of the dental prosthesis (sometimes also referred to as a prosthesis for dental applications) include dentures, inlays, onlays, crowns, connected crowns, bridges (bridges are also referred to as "pontic bridges" or "crown bridges"), fiber cores, and superstructures in implant treatment. A crown and a bridge are preferred, and a bridge is more preferred. The bridge may be a bridge of three or more units, or a bridge of up to 14 units. The bridge is preferably a three- or four-unit bridge, and more preferably a three-unit bridge. For example, as shown in FIGS. 5 and 6, the three-unit bridge has an abutment portion 5, a connecting portion 6, and a pontic portion 7. When no tooth is missing, but a tooth is loose (moves) due to periodontal disease, for example, the dental prosthesis of the present invention may be not only a bridge but also a prosthesis in which two or more crowns are connected (connected crowns) to firmly bond the adjacent teeth.

The three-unit bridge obtained by milling from the composite material for dental milling of the present invention has a fracture strength of typically 3000 N or more, preferably 4000 N or more, and more preferably 5000 N or more. Setting the fracture strength in the above-mentioned ranges can prevent a dental prosthesis (three-unit bridge) from being fractured by occlusal pressure during clinical use. The lower limit is preferably high, when considering the use as a bridge. The upper limit of the fracture strength is not specifically limited, and may be, for example, 8000 N or less, preferably 7000 N or less, and more preferably 6000 N or less. More specifically, the fracture strength of the three-unit bridge obtained by milling from the composite material for dental milling of the present invention is 3000 N or more and 8000 N or less, preferably 4000 N or more and 7000 N or less, and more preferably 5000 N or more and 6000 N or less.

Therefore, the composite material for dental milling of the present invention can be preferably used as a bridge, and particularly a three-unit bridge.

The method of measuring the fracture strength is described in detail in the Examples below.

Milling is preferably performed using a dental CAD/CAM system (apparatus). The dental CAD/CAM system (apparatus) may be a known apparatus. When the dental prosthesis is produced using a CAD/CAM system (apparatus), it can be produced by only a single milling operation. This allows the dental prosthesis to be produced more efficiently in a shorter time and with higher precision than conventionally produced manually. When the dental prosthesis is, for example, a bridge, the composite material for dental milling is preferably milled so that the first member (A) constitutes the connecting portion.

The dental prosthesis thus obtained from the composite material for dental milling of the present invention has a three-point bending strength required for a dental bridge and a three-point bending elastic modulus similar to those of tooth substances. A dental prosthesis including both a frame portion and a crown portion, such as a dental bridge, can be produced from the composite material for dental milling of the present invention by a single milling operation.

Examples

Hereinafter, the present invention will be described in more detail with examples; however, the technical scope of the present invention is not limited to these examples.

The methods for measuring various physical properties in the present invention are as follows.

(1) Three-Point Bending Strength

Specimens were cut out from the first member (core material) or the second member (coating material) obtained in each reference example and from the composite material for dental milling obtained in each example or the like, using a diamond cutting wheel in a precision cutting machine. The dimension of each specimen was set to a height (thickness) of 0.75 to 3.2 mm, based on JIS T 6526. The prepared specimen was subjected to a three-point bending test based on the measurement method according to JIS T 6526, to measure the three-point bending strength.

Specifically, the surface of the specimen was polished with waterproof abrasive paper (P2500) and then immersed in distilled water at 37°C for 24 hours, and the specimen was subjected to the three-point bending test using a compact universal testing machine (EZ-Graph, manufactured by Shimadzu Corporation) at a crosshead speed of 1 mm/min and a support span of 32 mm, to measure the three-point bending strength.

(2) Three-Point Bending Elastic Modulus

The three-point bending elastic modulus was determined from a slope of 25 to 50% of the maximum point test force (N) in a stress-displacement curve obtained in measurement of the three-point bending strength in the three-point bending test.

In each reference example or the like, four specimens were prepared and subjected to the three-point bending test, and the average value of the four measured values was determined as each of the three-point bending strength and the three-point bending elastic modulus.

(3) Density

Density was measured according to ISO 17304:2013. First, a mass ($m_1$) of a sample (diameter 15 mm, thickness 0.8 mm) was measured using an electronic balance (AUX120, manufactured by Shimadzu Corporation). Next, the sample was submerged in an aqueous sodium lauryl sulfate solution, and a mass ($m_2$) of the sample when left standing was measured using the electronic balance. Then, the density ($\rho$) of the sample was calculated using the equation (1) below. In the equation, $\rho_0$ is the density of the aqueous sodium lauryl sulfate solution.

$$\rho = (m_1 \times \rho_0)/(m_1 - m_2)$$

(4) Fracture Strength

[Three-Unit Bridge Fracture Test]

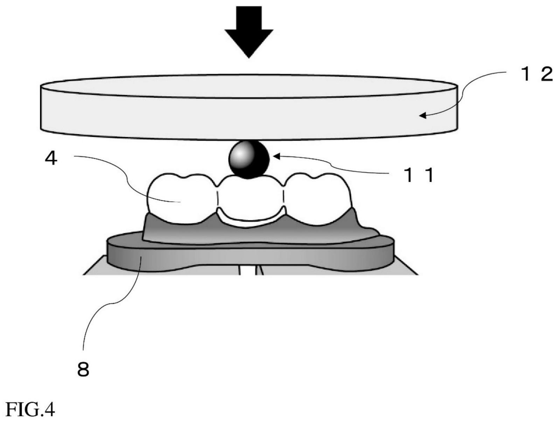

FIG. 4 shows a schematic diagram for explaining a three-unit bridge fracture test. A three-unit bridge 4 was left standing in distilled water at 37°C for 24 hours, and then a static fracture test was performed, as shown in FIG. 4, by placing a stainless steel ball 11 with a diameter of 8.0 mm on the occlusal surface of a pontic portion (middle tooth), and applying a load with a jig 12 for pressurization, using a compact universal testing machine (EZ-Graph, manufactured by Shimadzu Corporation), at a crosshead speed of 0.5 mm/min. Three specimens were prepared, and the average value of the three measured values was determined as the fracture strength.

1. Production Examples of First Member (A) (Core Material) and Evaluation of Physical Properties

Various components used in the first member (A) (core material) are as follows.

(C1) Polyfunctional (meth)acrylate-based polymerizable monomer

- UDMA: 2,2,4-trimethylhexamethylene bis(2-carbamoyloxyethyl) dimethacrylate (NF-501 urethane dimethacrylate, manufactured by Mitsubishi Chemical Corporation)
- Bis-EMA: ethoxylated bisphenol A dimethacrylate (BPE-80N, manufactured by Shin-Nakamura Chemical Co., Ltd.)

(C2) Low-viscosity polymerizable monomer

- MAA: methacrylic acid (MAA, manufactured by Mitsubishi Chemical Corporation, viscosity: less than 0.3 mPa•s (31°C))
- DEGDMA: diethylene glycol dimethacrylate (2G, manufactured by Shin-Nakamura Chemical Co., Ltd., viscosity: 3 mPa•s (31°C))
- TEGDMA: triethylene glycol dimethacrylate (3G, manufactured by Shin-Nakamura Chemical Co., Ltd., viscosity: 5 mPa•s (31°C))

The viscosity was measured using an SV viscometer.

(D) Glass fiber

- Glass fiber woven fabric (1) (ATG26100 treated tape, manufactured by Sakai Group)

     Single fiber diameter: 9 μm
     Thickness: 0.26 mm
     Mass: 23.9 g/m or more
     Basis weight: 237 g/m$^2$
     Weave: plain weave

- Glass fiber woven fabric (2) (#2116 (silane treated), manufactured by Allte Cloth Corporation)

     Single fiber diameter: 7 μm
     Thickness: 0.1 mm
     Basis weight: 104 g/m$^2$
     Weave: plain weave

Other Additives

(G) Polymerization initiator

- BPO: benzoyl peroxide (benzoyl peroxide, manufactured by Kishida Chemical Co., Ltd.)
- Coloring pigments: ferric oxide (red pigment), isoindolinone (yellow pigment), and titanium dioxide (white pigment) (manufactured by Dainichiseika Color & Chemicals Mfg. Co., Ltd.)

Reference Example 1 (First Member (Core Material) 1)

70 parts by mass of the component (C1) (UDMA), 30 parts by mass of the component (C2) (MAA), and 0.1 parts by mass of the component (G) (BPO) were mixed and stirred in a planetary centrifugal mixer (THINKY mixer, manufactured by Thinky Corporation) to give a polymerizable monomer-containing composition. As the glass fiber (D), 90 sheets of the glass fiber woven fabric (1) with a size of 100 mm per side were laminated and placed in the cavity portion of a mold; then the polymerizable monomer-containing composition was poured into the mold, and vacuum degassed and impregnated in a vacuum oven (VOS-451SD, manufactured by Tokyo Rikakikai Co., Ltd.). Then, the polymerizable monomer-containing composition was polymerized by being maintained in a convection oven (DRM620TD, manufactured by Advantec Co., Ltd.) at 150°C for 4 hours to give a composite material 1. The composite material 1 was cut into a dimension of 3.2 mm in height, 4.2 mm in width, and 34 mm in length using a diamond cutting wheel in a precision cutting machine to give a first member (core material) of Reference Example 1.

Reference Example 2 (First Member (Core Material) 2)

A composite material 2 was obtained as in Reference Example 1, except that the glass fiber (D) was changed from the glass fiber woven fabric (1) to the glass fiber woven fabric (2), and the number of laminated sheets of the glass fiber woven fabric (2) was 220. The composite material 2 was cut into a dimension of 3.2 mm in height, 4.2 mm in width, and 34 mm in length to give a first member (core material) of Reference Example 2.

Reference Example 3 (First Member (Core Material) 3)

A composite material 3 was obtained as in Reference Example 1, except that 0.0016 parts by mass of the red pigment, 0.0024 parts by mass of the yellow pigment, and 0.325 parts by mass of the white pigment were added to the polymerizable monomer-containing composition. The composite material 3 was cut into a dimension of 0.75 mm in height, 4.2 mm in width, and 34 mm in length to give a first member (core material) of Reference Example 3.

Reference Examples 4 to 8 (First Members (Core Materials) 4 to 8)

A composite material 3 produced as in Reference Example 3 was cut into the dimensions as shown in Table 1 to give first members (core materials) of Reference Examples 4 to 8.

Reference Example 9 (First Member (Core Material) 9)

A composite material 4 was obtained as in Reference Example 1, except that the coloring pigments were added to the polymerizable monomer-containing composition as in Reference Example 3, and that the glass fiber (D) was changed from the glass fiber woven fabric (1) to the glass fiber woven fabric (2), and the number of laminated sheets of the glass fiber woven fabric was 220. The composite material 4 was cut into a dimension of 3.2 mm in height, 4.2 mm in width, and 34 mm in length to give a first member (core material) of Reference Example 9.

Reference Example 10 (First Member (Core Material) 10)

A composite material 5 was obtained as in Reference Example 3, except that 35 sheets of the glass fiber woven fabric (1) with a size of 100 mm per side and a thickness of 0.26 mm were laminated, and 110 sheets of the glass fiber woven fabric (2) with a size of 100 mm per side and a thickness of 0.1 mm were laminated thereon and placed in the cavity portion of a mold. The composite material 5 was cut into a dimension of 3.2 mm in height, 4.2 mm in width, and 34 mm in length so that the boundary between the glass fiber woven fabric (1) and the glass fiber woven fabric (2) was centrally positioned in the height direction to give a first member (core material) of Reference Example 10.

Comparative Reference Example 1 (First Member (Core Material) 11)

A composite material 3 produced as in Reference Example 3 was cut into a dimension of 0.5 mm in height, 4.2 mm in width, and 34 mm in length to give a first member (core material) of Comparative Reference Example 1.

Comparative Reference Example 2 (First Member (Core Material) 12)

Trinia (manufactured by Bicon, Inc., block type, 40 mm × 19 mm × 15 mm, ivory (hereinafter referred to as the commercial product A)), which is a composite material for dental milling containing a curable resin and a glass fiber woven fabric, was used as the first member (core material)), and cut into a dimension of 3.2 mm in height, 4.2 mm in width, and 34 mm in length to give a first member (core material) of Comparative Reference Example 2.

Three-point bending strength was measured according to the method described above, for Reference Examples 1 to 10 and Comparative Reference Examples 1 and 2. The composition, dimensions, and results for each reference example or comparative reference example are shown in Table 1 below.

[Table 1]

| Table 1 | First Member (A) | | | | | | | Three-Point Bending Test |
|---|---|---|---|---|---|---|---|---|
| | Glass Fiber (D) | Presence of Pigment | Height (mm) | Width (mm) | Length (mm) | Cross-Sectional Area (mm$^2$) | Volume (mm$^3$) | Three-Point Bending Strength (MPa) |
| Ref. Ex. 1 | (1) | No | 3.2 | 4.2 | 34 | 13.4 | 457.0 | 946 |
| Ref. Ex. 2 | (2) | No | 3.2 | 4.2 | 34 | 13.4 | 457.0 | 799 |
| Ref. Ex. 3 | (1) | Yes | 0.75 | 4.2 | 34 | 3.2 | 107.1 | 608 |
| Ref. Ex. 4 | (1) | Yes | 1.0 | 4.2 | 34 | 4.2 | 142.8 | 709 |
| Ref. Ex. 5 | (1) | Yes | 1.5 | 4.2 | 34 | 6.3 | 214.2 | 781 |
| Ref. Ex. 6 | (1) | Yes | 2.0 | 4.2 | 34 | 8.4 | 285.6 | 808 |
| Ref. Ex. 7 | (1) | Yes | 2.5 | 4.2 | 34 | 10.5 | 357.0 | 786 |
| Ref. Ex. 8 | (1) | Yes | 3.2 | 4.2 | 34 | 13.4 | 457.0 | 779 |
| Ref. Ex. 9 | (2) | Yes | 3.2 | 4.2 | 34 | 13.4 | 457.0 | 631 |
| Ref. Ex. 10 | (1) + (2) | Yes | 3.2 | 4.2 | 34 | 13.4 | 457.0 | 570 |
| Comp. Ref. Ex. 1 | (1) | Yes | 0.5 | 4.2 | 34 | 2.1 | 71.4 | 441 |
| Comp. Ref. Ex. 2 | Commercial Product A | | 3.2 | 4.2 | 34 | 13.4 | 457.0 | 396 |

Table 1 shows that the first members (core materials) of Reference Examples 1 to 10, which had a cross-sectional area of 3 to 14 mm$^2$, had a three-point bending strength of 500 MPa or more.

2. Production Examples of Second Member (B) and Evaluation of Physical Properties Various components used in the second member (B) are as follows.

(E1) (Meth)acrylate-based polymerizable monomer

- UDMA: 2,2,4-trimethylhexamethylene bis(2-carbamoyloxyethyl) dimethacrylate (NF-501 urethane dimethacrylate, manufactured by Mitsubishi Chemical Corporation)
- DEGDMA: diethylene glycol dimethacrylate (2G, manufactured by Shin-Nakamura Chemical Co., Ltd., viscosity: 3 mPa•s (31°C))

The viscosity was measured using an SV viscometer.

(F) Inorganic filler

Inorganic fillers (f1), (f2), and (f3) prepared by the following methods were used.

Preparation of Composite Metal Oxide Filler (f1)

10 parts by mass of aluminum nitrate nonahydrate ($Al(NO_3)_3•9H_2O$) and 15 parts by mass of AP-1 (denatured ethanol, AP-1, manufactured by Japan Alcohol Trading Co., Ltd., ethanol 87 wt%, isopropyl alcohol 13 wt%) were mixed and dissolved. Next, 118 parts by mass of Zircosol ZN (aqueous zirconium nitrate solution, manufactured by Daiichi Kigenso Kagaku Kogyo Co., Ltd., $ZrO_2$ content = 25 wt%) was added to the resulting solution, and 280 parts by mass of MS51 (methyl silicate oligomer, manufactured by Mitsubishi Chemical Corporation, $SiO_2$ content = 52 wt%) and 430 parts by mass of distilled water were added. Then, the resulting mixture was mixed with a stirrer for 60 minutes to prepare a transparent and uniform raw material mixture solution. Next, 127 parts by mass of a two-fold dilution of an aqueous ammonia solution (for example, manufactured by Nacalai Tesque, Inc., $NH_3 = 28\%$) was added to the previously prepared raw material mixture solution while stirring, which caused co-precipitation and gelation of the mixture into a jelly-like form. The gel was taken out and dried at 100°C to remove excess ammonia, water, and solvent to give a dry gel. The dry gel was washed with water and filtered to remove by-product ammonium nitrate and dried again. The dry gel needs to be sufficiently washed with water because, if a large amount of ammonium nitrate remains, the gas may be generated during firing, with the risk of explosion.

100 parts by mass of the dry gel was dispersed in 250 parts by mass of AP-1, and pulverized for 4 hours using a bead mill (MULUTI-LABO, manufactured by Shinmaru Enterprises Corporation) filled with a prescribed amount of zirconia balls with a diameter of 0.65 mm to prepare a slurry. The particle diameter and the particle size distribution of the slurry were measured; the slurry had an average particle diameter of 0.6 $\mu$m, and the particle diameter was not pulverized to less than 0.2 $\mu$m.

The slurry was collected and dried to remove the solvent. The particles at this stage correspond to the primary particles of the finished filler. Next, the pulverized and dried gel was processed with a jet mill (100AFG/50ATP, manufactured by Hosokawa Micron Corporation) to give a gel powder with an average particle diameter of about 20 $\mu$m. The gel was placed in a dish made of alumina and heated to 1100°C at a heating rate of 270°C per hour in an electric furnace, held at the same temperature for 3.5 hours, and then taken out of the furnace and allowed to cool to give a white powder.

The fired gel was pulverized with the above-mentioned jet mill to give a composite metal oxide filler containing $SiO_2$, $ZrO_2$, and $Al_2O_3$. The composite metal oxide had an average particle diameter of 5.5 $\mu$m (10% D: 0.4 $\mu$m, 50% D: 10.1 $\mu$m, 90% D: 26.9 $\mu$m) and was found to be a polydisperse system having a broad distribution in the range of about 0.5 to about 50 $\mu$m.

100 parts by mass of the composite metal oxide filler was suspended in 200 parts by mass of an alcohol solvent (AP-1), and 9 parts by mass of $\gamma$-MPTS (TSL-8370, manufactured by Momentive Performance Materials Japan LLC) was added, and then the mixture was subjected to ultrasonic dispersion for 1 hour. Then, the solvent was removed with an evaporator, and then the mixture was dried under reduced pressure at 80°C for 2 hours and dried under reduced pressure at 110°C for 1 hour to give a composite metal oxide filler surface-treated with a silane coupling agent.

Preparation of Inorganic Filler Containing Fluoride (f2)

100 parts by mass of a dental glass filler (G018-090 (UF0.7), manufactured by Schott Japan Corporation) with an average particle diameter of 0.7 $\mu$m that releases fluoride ions and 2 parts by mass of $\gamma$-MPTS were added into 200 parts by mass of an alcohol solvent (AP-1), and the mixture was subjected to ultrasonic dispersion for 1 hour. Then, the alcohol solvent was removed with an evaporator, and then the mixture was dried under reduced pressure at 80°C for 2 hours and dried under reduced pressure at 110°C for 1 hour to give a fluorine filler surface-treated with a silane coupling agent.

Preparation of Ultrafine $SiO_2$ Filler (f3)

10 parts by mass of $\gamma$-MPTS was added into 100 parts by mass of a colloidal silica filler solution with an average particle diameter of 15 nm (MEK-ST 100, manufactured by Nissan Chemical Corporation, containing 30% by mass of $SiO_2$ in methyl ethyl ketone), which is an ultrafine $SiO_2$ filler, and the mixture was subjected to ultrasonic dispersion for 1 hour. Then, 60 parts by mass of a polymerizable monomer (UDMA) was added, and then the solvent was removed with an evaporator to give UDMA containing 30% by mass of the ultrafine $SiO_2$ filler surface-treated with the silane coupling agent. A composite material for dental milling was produced by mixing an appropriate amount of UDMA containing the ultrafine $SiO_2$ filler.

<Other Additives>

- Polymerization initiator: BPO (benzoyl peroxide)
- Coloring pigments: opacifying agent (zirconium oxide ($ZrO_2$)), black pigment (ferrosoferric oxide ($Fe_3O_4$)), red pigment (ferric oxide ($Fe_2O_3$)), and yellow pigment

(isoindolinone)

Reference Example 11 (Second Member (Coating Material) 1)

A mixed polymerizable monomer (E) of UDMA and DEGDMA obtained by adding 1% by mass of a polymerization initiator (BPO) to the polymerizable monomers was mixed with UDMA containing the composite metal oxide filler (f1), the fluorine filler (f2), and the ultrafine $SiO_2$ filler (f3), to produce a paste containing 29% by mass of the polymerizable monomer (E) (UDMA/DEGDMA = 80/20 in terms of mass ratio), 48% by mass of the composite metal oxide filler (f1), 21% by mass of the fluorine filler (f2), and 3% by mass of the ultrafine $SiO_2$ filler (f3). Subsequently, the paste was uniformly kneaded and degassed under reduced pressure to give a coating material composition. A mold was filled with the resulting coating material composition, which was polymerized and cured, and then the cured product was cut into a dimension of 3.2 mm in height, 4.2 mm in width, and 34 mm in length to give a second member (coating material) of Reference Example 11.

Reference Example 12 (Second Member (Coating Material) 2)

Instead of the coating material composition, a mold was filled with a hard resin for teeth crowns, TWiNY (manufactured by YAMAKIN Co., Ltd., DA3 Flow (hereinafter, referred to as the commercial product B)), and the resin was polymerized and cured by performing photo irradiation for 90 seconds with a photopolymerization apparatus (LED Cure Master, manufactured by DENKEN-HIGHDENTAL Co., Ltd.). Then, the cured product was heat-treated at 110°C for 10 minutes in a heat polymerization apparatus, and then cut into a dimension of 3.2 mm in height, 4.2 mm in width, and 34 mm in length to give a second member (coating material) of Reference Example 12.

Reference Example 13 (Second Member (Coating Material) 3)

Instead of the coating material composition, a mold was filled with a hard resin for teeth crowns, Luna-Wing (manufactured by YAMAKIN Co., Ltd., DA3 Flow (hereinafter, referred to as the commercial product C)), the resin was polymerized and cured by performing photo irradiation for 90 seconds with a photopolymerization apparatus (LED Cure Master, manufactured by DENKEN-HIGHDENTAL Co., Ltd.), and the cured product was cut into a dimension of 3.2 mm in height, 4.2 mm in width, and 34 mm in length to give a second member (coating material) of Reference Example 13.

Three-point bending strength was measured according to the method described above, for Reference Examples 11 to 13. The materials and dimensions, and results for each reference example are shown in Table 2 below.

[Table 2]

| Table 2 | Second Member (B) | | | | | | Three-Point Bending Test |
|---|---|---|---|---|---|---|---|
| | Material | Height (mm) | Width (mm) | Length (mm) | Cross-Sectional Area (mm²) | Volume (mm³) | Three-Point Bending Strength (MPa) |
| Ref. Ex. 11 | Second Member Composition | 3.2 | 4.2 | 34 | 13.4 | 457.0 | 209 |
| Ref. Ex. 12 | Commercial Product B | 3.2 | 4.2 | 34 | 13.4 | 457.0 | 190 |
| Ref. Ex. 13 | Commercial Product C | 3.2 | 4.2 | 34 | 13.4 | 457.0 | 124 |

Table 2 shows that the second members (coating materials) of Reference Examples 11 to 13 had a three-point bending strength of less than 500 MPa.

3. Production Examples of Composite Material for Dental Milling and Evaluation of Physical Properties

Example 1

70 parts by mass of the component (C1) (UDMA), 30 parts by mass of the component (C2) (MAA), and 0.1 parts by mass of the component (G) (BPO) were mixed and stirred in a planetary centrifugal mixer (THINKY mixer, manufactured by Thinky Corporation) to give a polymerizable monomer-containing composition. Ninety sheets of the glass fiber woven fabric (1) with a size of 100 mm per side were laminated and placed in the cavity portion of a mold; then the polymerizable monomer-containing composition was poured into the mold, and vacuum degassed and impregnated in a vacuum oven (VOS-451SD, manufactured by Tokyo Rikakikai Co., Ltd.). Then, the polymerizable monomer-containing composition was polymerized by being maintained in a convection oven (DRM620TD, manufactured by Advantec Co., Ltd.) at 150°C for 4 hours to give a first member. The first member was cut into a dimension of 0.75 mm in height, 4.2 mm in width, and 34 mm in length.

A mixed polymerizable monomer (E) of UDMA and DEGDMA obtained by adding 1% by mass of a polymerization initiator (BPO) to the polymerizable monomers was mixed with UDMA containing the composite metal oxide filler (f1), the fluorine filler (f2), and the ultrafine $SiO_2$ filler (f3), to prepare a paste containing 29% by mass of the polymerizable monomer (E) (UDMA/DEGDMA = 80/20 in terms of mass ratio), 48% by mass of the composite metal oxide filler (f1), 21% by mass of the fluorine filler (f2), and 3% by mass of the ultrafine $SiO_2$ filler (f3). Subsequently, the paste was uniformly kneaded and degassed under reduced pressure to give a second member composition.

MultiPrimer Liquid (manufactured by YAMAKIN Co., Ltd.) was applied using a flat brush over the interface of the first member with the second member and dried for 2 minutes, and then the first member was placed in a mold (36 mm in length × 40 mm in width × 22 mm in thickness). The mold was then filled with the second member composition, which was maintained at 160°C for 2 hours to be polymerized and cured. The cured product was cut into a dimension of 3.2 mm in height, 4.2 mm in width, and 34 mm in length, including the first member, thus giving a two layer-type composite material for dental milling including the first member and the second member, as shown in FIG. 1.

Examples 2 to 6

Two layer-type composite materials for dental milling were obtained as in Example 1, except that each of the first members was cut into the dimension as shown in Table 3.

Example 7

A two layer-type composite material for dental milling was obtained as in Example 1, except that 0.0016 parts by mass of the red pigment, 0.0024 parts by mass of the yellow pigment, and 0.325 parts by mass of the white pigment were added to the polymerizable monomer-containing composition, and that the first member was cut into the dimension as shown in Table 3.

Examples 8 to 11

Two layer-type composite materials for dental milling were obtained as in Example 7, except that each of the first members was cut into the dimension as shown in Table 3.

Example 12

A two layer-type composite material for dental milling was obtained as in Example 1, except that 0.0016 parts by mass of the red pigment, 0.0024 parts by mass of the yellow pigment, and 0.325 parts by mass of the white pigment were added to the polymerizable monomer-containing composition; the glass fiber (D) was changed from the glass fiber woven fabric (1) to the glass fiber woven fabric (2), and the number of laminated sheets thereof was changed to 220; and the first member was cut into the dimension as shown in Table 3.

Example 13

A first member was produced as in Example 1, except that 0.0016 parts by mass of the red pigment, 0.0024 parts by mass of the yellow pigment, and 0.325 parts by mass of the white pigment were added to the polymerizable monomer-containing composition. Then, the first member was placed in a mold. The commercial product B (TWiNY Flow, manufactured by YAMAKIN Co., Ltd.) was used as the material of the coating material. The mold was filled with the commercial product B, which was polymerized and cured by performing photo irradiation for 90 seconds with a photopolymerization apparatus (LED Cure Master, manufactured by DENKEN-HIGHDENTAL Co., Ltd.). Then, the cured product was heat-treated at 110°C for 10 minutes in a heat polymerization apparatus, to give a two layer-type composite material for dental milling.

Example 14

A two layer-type composite material for dental milling was obtained as in Example 13, except that the first member was cut into the dimension as shown in Table 3.

Example 15

70 parts by mass of the component (C1) (UDMA), 30 parts by mass of the component (C2) (MAA), and 0.1 parts by mass of the component (G) (BPO) were blended with 0.0016 parts by mass of the red pigment, 0.0024 parts by mass of the yellow pigment, and 0.325 parts by mass of the white pigment, and then stirred in a planetary centrifugal mixer (THINKY mixer, manufactured by Thinky Corporation) to give a polymerizable monomer-containing composition. Thirty-five sheets of the glass fiber woven fabric (1) with a size of 100 mm per side and a thickness of 0.26 mm were laminated, and 110 sheets of the glass fiber woven fabric (2) with a size of 100 mm per side and a thickness of 0.1 mm were laminated thereon and placed in the cavity portion of a mold; then the polymerizable monomer-containing composition was poured into the mold, and vacuum degassed and impregnated in a vacuum oven (VOS-451SD, manufactured by Tokyo Rikakikai Co., Ltd.). Then, the polymerizable monomer-containing composition was polymerized by being maintained in a convection oven (DRM620TD, manufactured by Advantec Co., Ltd.) at 150°C for 4 hours to give a first member. The first member was cut into the dimension as shown in Table 3 so that the boundary between the glass fiber woven fabric (1) and the glass fiber woven fabric (2) was centrally positioned in the height direction.

Then, MultiPrimer Liquid (manufactured by YAMAKIN Co., Ltd.) was applied using a flat brush over the surface of the upper layer (the layer containing the glass fiber woven fabric (2) as the glass fiber (D)) of the first member having a two-layer structure, and dried for 2 minutes. The first member was then placed in a mold (36 mm in length × 40 mm in width × 22 mm in thickness), and the mold was filled with the second member composition, which was maintained at 160°C for 2 hours to be polymerized and cured, so that the second member composition was laminated on the first member. The cured product was cut into a dimension of 3.2 mm in height, 4.2 mm in width, and 34 mm in length, including the first member, thus giving a three layer-type composite material for dental milling including the first member (in two layers) and the second member, as shown in FIG. 2.

Example 16

A first member was produced as in Example 1, except that 0.0016 parts by mass of the red pigment, 0.0024 parts by mass of the yellow pigment, and 0.325 parts by mass of the white pigment were added to the polymerizable monomer-containing composition, and that the first member was cut into the dimension as shown in Table 3. Then, the first member was placed in a mold. The commercial product C (Luna-Wing Flow, manufactured by YAMAKIN Co., Ltd.) was used as the material of the second member. The mold was filled with the commercial product C, which was polymerized and cured by performing photoirradiation for 90 seconds with a photopolymerization apparatus (LED Cure Master, manufactured by DENKEN-HIGHDENTAL Co., Ltd.), to give a two layer-type composite material for dental milling.

Example 17

A first member (core material) was produced as in Example 1 and cut into a dimension of 3 mm in height, 3.5 mm in width, and 34 mm in length, and MultiPrimer Liquid (manufactured by YAMAKIN Co., Ltd.) was applied thereto and dried for 2 minutes. Then, the first member (core material) was placed in the center of a cavity portion (3.2 mm in height, 4.2 mm in width, and 34 mm in length) of a mold, and the cavity portion of the mold was filled with the commercial product B as the material of the second member (coating material), which was then polymerized by being maintained at 160°C for 2 hours. This resulted in a composite material for dental milling of the coated core material type, including the core material and the coating material, having the structure as shown in FIG. 3. Here, a front view for illustrating the composite material for dental milling of the coated core material type is shown in FIG. 3a, and a cross-sectional view along the line A-A' of the front view is shown in FIG. 3b. The coating material is coated at a thickness of 0.2 mm on each of the upper and lower surfaces in the height direction of the core material, and coated at a thickness of 0.7 mm on each of the left and right surfaces in the width direction of the core material.

Example 18

A composite material for dental milling of the coated core material type as shown in FIG. 3 was obtained as in Example 17, except that the glass fiber (D) was changed from the glass fiber woven fabric (1) to the glass fiber woven fabric (2), and the number of laminated sheets thereof was changed to 220.

Comparative Example 1

A two layer-type composite material for dental milling was produced as in Example 1, except that the first member was cut into the dimension as shown in Table 3.

Comparative Example 2

A two layer-type composite material for dental milling was produced as in Example 7, except that the first member was cut into the dimension as shown in Table 3.

Comparative Examples 3 and 4

Two layer-type composite materials for dental milling were produced as in Example 1, except that the commercial product A as each of the first members was cut into the dimension as shown in Table 3.

Three-point bending strength and three-point bending elastic modulus were measured according to the methods described above, for Examples 1 to 18 and Comparative Examples 1 to 4. Each of the specimens of Examples 1 to 16 was placed so that the second member faced up (load plunger side), and subjected to measurement. The materials and dimensions, and results for each example or the like are shown in Table 3 below.

[Table 3]

| Table 3 | First Member (A) | | | | | | | Second Member (B) | | | | | | Three-Point Bending Test | |
|---|---|---|---|---|---|---|---|---|---|---|---|---|---|---|---|
| | Glass Fiber (D) | Presence of Pigment | Height (mm) | Width (mm) | Length (mm) | Cross-Sectional Area (mm²) | Volume (mm³) | Material | Height (mm) | Width (mm) | Length (mm) | Cross-Sectional Area (mm²) | Volume (mm³) | Three-Point Bending Strength (MPa) | Three-Point Bending Elastic Modulus (GPa) |
| Ex. 1 | (1) | No | 0.75 | 4.2 | 34 | 3.2 | 107.1 | Second Member Composition | 2.45 | 4.2 | 34 | 10.3 | 349.9 | 548 | 18 |
| Ex. 2 | (1) | No | 1.0 | 4.2 | 34 | 4.2 | 142.8 | Second Member Composition | 2.2 | 4.2 | 34 | 9.2 | 314.2 | 753 | 18 |
| Ex. 3 | (1) | No | 1.5 | 4.2 | 34 | 6.3 | 214.2 | Second Member Composition | 1.7 | 4.2 | 34 | 7.1 | 242.8 | 748 | 21 |
| Ex. 4 | (1) | No | 2.0 | 4.2 | 34 | 8.4 | 285.6 | Second Member Composition | 1.2 | 4.2 | 34 | 5.0 | 171.4 | 726 | 21 |
| Ex. 5 | (1) | No | 2.5 | 4.2 | 34 | 10.5 | 357.0 | Second Member Composition | 0.7 | 4.2 | 34 | 2.9 | 100.0 | 820 | 24 |
| Ex. 6 | (1) | No | 3.0 | 4.2 | 34 | 12.6 | 428.4 | Second Member Composition | 0.2 | 4.2 | 34 | 0.8 | 28.6 | 885 | 32 |
| Ex. 7 | (1) | Yes | 1.0 | 4.2 | 34 | 4.2 | 142.8 | Second Member Composition | 2.2 | 4.2 | 34 | 9.2 | 314.2 | 525 | 15 |
| Ex. 8 | (1) | Yes | 1.5 | 4.2 | 34 | 6.3 | 214.2 | Second Member Composition | 1.7 | 4.2 | 34 | 7.1 | 242.8 | 555 | 16 |

(continued)

| Table 3 | First Member (A) | | | | | | | Second Member (B) | | | | | | Three-Point Bending Test | |
|---|---|---|---|---|---|---|---|---|---|---|---|---|---|---|---|
| | Glass Fiber (D) | Presence of Pigment | Height (mm) | Width (mm) | Length (mm) | Cross-Sectional Area (mm²) | Volume (mm³) | Material | Height (mm) | Width (mm) | Length (mm) | Cross-Sectional Area (mm²) | Volume (mm³) | Three-Point Bending Strength (MPa) | Three-Point Bending Elastic Modulus (GPa) |
| Ex. 9 | (1) | Yes | 2.0 | 4.2 | 34 | 8.4 | 285.6 | Second Member Composition | 1.2 | 4.2 | 34 | 5.0 | 171.4 | 588 | 18 |
| Ex. 10 | (1) | Yes | 2.5 | 4.2 | 34 | 10.5 | 357.0 | Second Member Composition | 0.7 | 4.2 | 34 | 2.9 | 100.0 | 623 | 21 |
| Ex. 11 | (1) | Yes | 3.0 | 4.2 | 34 | 12.6 | 428.4 | Second Member Composition | 0.2 | 4.2 | 34 | 0.8 | 28.6 | 703 | 26 |
| Ex. 12 | (2) | Yes | 3.0 | 4.2 | 34 | 12.6 | 428.4 | Second Member Composition | 0.2 | 4.2 | 34 | 0.8 | 28.6 | 560 | 23 |
| Ex. 13 | (1) | Yes | 1.0 | 4.2 | 34 | 4.2 | 142.8 | Commercial Product B | 2.2 | 4.2 | 34 | 9.2 | 314.2 | 512 | 15 |
| Ex. 14 | (1) | Yes | 2.0 | 4.2 | 34 | 8.4 | 285.6 | Commercial Product B | 1.2 | 4.2 | 34 | 5.0 | 171.4 | 521 | 16 |
| Ex. 15 | (1) + (2) | Yes | 3.0 | 4.2 | 34 | 12.6 | 428.4 | Second Member Composition | 0.2 | 4.2 | 34 | 0.8 | 28.6 | 505 | 18 |
| Ex. 16 | (1) | Yes | 3.0 | 4.2 | 34 | 12.6 | 428.4 | Commercial Product C | 0.2 | 4.2 | 34 | 0.8 | 28.6 | 720 | 26 |
| Ex. 17 | (1) | No | 3.0 | 3.5 | 34 | 10.5 | 357.0 | Commercial Product B | - | - | 34 | 2.9 | 100.0 | 693 | 20 |
| Ex. 18 | (2) | No | 3.0 | 3.5 | 34 | 10.5 | 357.0 | Commercial Product B | - | - | 34 | 2.9 | 100.0 | 580 | 21 |

| Table 3 | Glass Fiber (D) | Presence of Pigment | First Member (A) | | | | | Second Member (B) | | | | | | Three-Point Bending Test | |
|---|---|---|---|---|---|---|---|---|---|---|---|---|---|---|---|
| | | | Height (mm) | Width (mm) | Length (mm) | Cross-Sectional Area (mm$^2$) | Volume (mm$^3$) | Material | Height (mm) | Width (mm) | Length (mm) | Cross-Sectional Area (mm$^2$) | Volume (mm$^3$) | Three-Point Bending Strength (MPa) | Three-Point Bending Elastic Modulus (GPa) |
| Comp. Ex. 1 | (1) | No | 0.5 | 4.2 | 34 | 2.1 | 71.4 | Second Member Composition | 2.7 | 4.2 | 34 | 11.3 | 385.6 | 343 | 18 |
| Comp. Ex. 2 | (1) | Yes | 0.5 | 4.2 | 34 | 2.1 | 71.4 | Second Member Composition | 2.7 | 4.2 | 34 | 11.3 | 385.6 | 308 | 14 |
| Comp. Ex. 3 | Commercial Product A | | 1.0 | 4.2 | 34 | 4.2 | 142.8 | Second Member Composition | 2.2 | 4.2 | 34 | 9.2 | 314.2 | 333 | 11 |
| Comp. Ex. 4 | Commercial Product A | | 3.0 | 4.2 | 34 | 12.6 | 428.4 | Second Member Composition | 0.2 | 4.2 | 34 | 0.8 | 28.6 | 384 | 13 |

Table 3 shows that, in Examples 1 to 18, the first members (core materials) had a cross-sectional area of 3 to 14 $mm^2$, and therefore, the three-point bending strength was 500 MPa or more, and the three-point bending elastic modulus was 15 to 32 GPa. The composite materials for dental milling of Examples 1 to 18 according to the present invention each had a three-point bending strength required for a dental bridge and a three-point bending elastic modulus similar to those of tooth substances.

In contrast, in Comparative Examples 1 and 2, the cross-sectional area was less than 3 $mm^2$, and therefore, the three-point bending strength of 500 MPa or more was not obtained.

In Comparative Examples 3 and 4, the three-point bending strength was 500 MPa or less, although the cross-sectional area was 3 to 14 $mm^2$. This is believed to be due to the low strength of the first member (core material), as shown in Comparative Reference Example 2 in Table 1.

Example 19

A first member (core material) was produced as in Example 1 and cut into a dimension of 2 mm in height, 3 mm in width, and 40 mm in length, and MultiPrimer Liquid (manufactured by YAMAKIN Co., Ltd.) was applied thereto and dried for 2 minutes. Then, the first member (core material) was placed in the center of a cavity portion (16 mm in height, 19 mm in width, and 40 mm in length) of a mold, and the cavity portion of the mold was filled with the coating material composition, which was then polymerized by being maintained at 160°C for 2 hours. This resulted in a composite material for dental milling of the coated core material type, including the core material and the coating material, as shown in FIG. 3. Then, the composite material for dental milling was cut into a plate shape of 16 mm in height, 19 mm in width, and 0.8 mm in length.

Comparative Examples 5 to 7

A commercial product D of a metal material (KZR-CAD titanium, manufactured by YAMAKIN Co., Ltd.), a commercial product E of a ceramic material (KZR-CAD zirconia SHT, manufactured by YAMAKIN Co., Ltd.), and a commercial product F of a precious metal material (PALLA Z 12-n, manufactured by YAMAKIN Co., Ltd.), which have been conventionally used in the production of dental prostheses such as three-unit bridges, were each processed into a plate shape of 16 mm in height, 19 mm in width, and 0.8 mm in length, and used as Comparative Examples 5 to 7, respectively.

Density was measured according to the method described above, for Example 19 and Comparative Examples 5 to 7. The results are shown in Table 4 below.

[Table 4]

| Table 4 | Material | Density (g/cm$^3$) |
|---|---|---|
| Ex. 19 | Composite Material for Dental Milling | 2.0 |
| Comp. Ex. 5 | Commercial Product D (Titanium Alloy) | 4.4 |
| Comp. Ex. 6 | Commercial Product E (Zirconia) | 6.1 |
| Comp. Ex. 7 | Commercial Product F (Gold-Silver-Palladium Alloy) | 11.0 |

Table 4 shows that Example 19 (composite material for dental milling of the present invention) had a density in the range of 1 to 4 g/cm$^3$. Thus, the composite material for dental milling of the present invention, which has a density in the range similar to that of human tooth substances, is less likely to cause loss of occlusal balance, and therefore, can possibly reduce health hazards (such as abrasion or fracture of teeth, temporomandibular disorders, stiff shoulders, and stress) caused by loss of occlusal balance.

In contrast, Comparative Examples 5 to 7 had a density of more than 4 g/cm$^3$. Therefore, the composite materials of Comparative Examples 5 to 7 may possibly cause health hazards due to loss of occlusal balance.

4. Production Examples of Prostheses (Bridges) and Evaluation of Physical Properties Example 20

A composite material for dental milling of the coated core material type (16 mm in height, 19 mm in width, and 40 mm in length), including a core material (2 mm in height, 2 mm in width, and 40 mm in length) in the center and a coating material therearound, was produced as in Example 18, except that the first member (core material) was produced as in Example 1 and cut into a bar shape of 2 mm in height, 2 mm in width, and 40 mm in length.

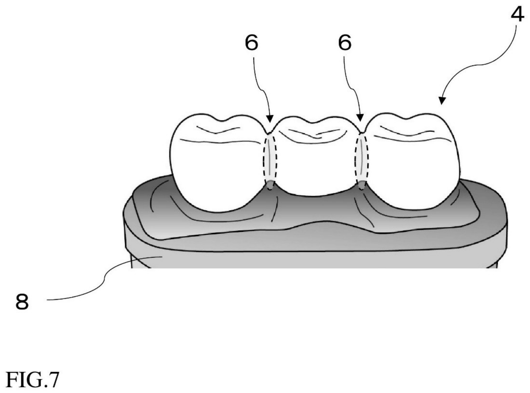

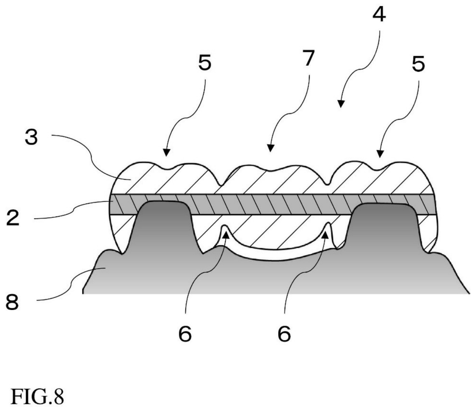

Then, the composite material for dental milling was produced into a specimen 4 in the shape of a three-unit bridge as shown in FIG. 6, using a CAD/CAM system. In the three-unit bridge 4 including the first member (core material) 2 and the second member (coating material) 3, the occlusal surface had a thickness of 2.0 mm or more, the axial surface had a thickness of 1.5 mm or more, the margin portion had a thickness of 0.8 mm or more, and the connecting portion 6 had a height of 3.6 mm or more and a width of 5.2 mm or more. The bonding surface of the three-unit bridge 4 was surface-treated by alumina sandblasting (particle diameter: 50 μm, pressure: 0.2 MPa), ultrasonically cleaned in ethanol, and dried. Then, the resulting specimen was bonded to a titanium base according to the method of using a dental adhesive resin cement (Superbond (registered trademark)), manufactured by Sun Medical Co., Ltd). A perspective view illustrating the specimen of Example 20 bonded to the base 8 is shown in FIG. 7, and a cross-sectional schematic view of FIG. 7 is shown in FIG. 8.

Examples 21 and 22

Composite materials for dental milling of the coated core material type were obtained as in Example 20, except that a core material produced as in Example 19 except that the glass fiber (D) was changed from the glass fiber woven fabric (1) to the glass fiber woven fabric (2), and that the number of laminated sheets of the glass fiber woven fabric (2) was changed to 220, was cut into the dimension as shown in Table 3.

Comparative Example 8 (Milled Frame + Milled Resin Teeth)

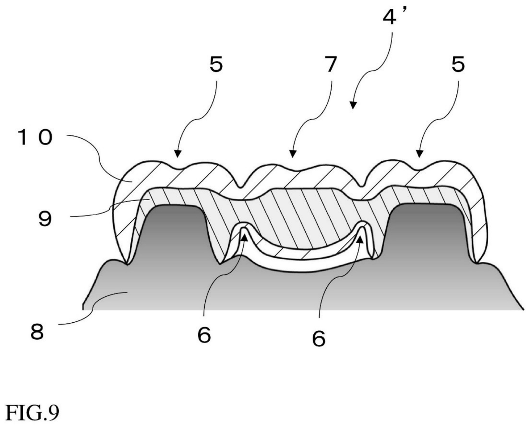

A composite material 9 was produced as in Reference Example 9 and cut into a block shape of 16 mm in height, 19 mm in width, and 40 mm in length. A milled frame was produced from the resulting block using a CAD/CAM system. The milled frame was milled so that the thickness of the occlusal surface was 1.0 mm or more, the thickness of the axial surface was 0.8 mm or more, and the height and width of the connecting portion were the dimensions as shown in Table 3 and more. The cross-sectional shape of the milled frame is indicated as 9 in FIG. 9.

A second member (coating material) 1 was produced as in Reference Example 11 and cut into a block shape of 16 mm in height, 19 mm in width, and 40 mm in length. Milled resin teeth were produced from the resulting block using a CAD/CAM system. The cross-sectional shape of the milled resin teeth is indicated as 10 in FIG. 9.

The bonding surface of the resulting milled resin teeth was surface-treated by alumina sandblasting (particle diameter: 50 μm, pressure: 0.2 MPa), ultrasonically cleaned in ethanol, and dried. Then, the milled resin teeth were bonded to the milled frame according to the method of using a dental adhesive resin cement (Superbond (registered trademark)), manufactured by Sun Medical Co., Ltd) to produce a three-unit bridge.

In the resulting three-unit bridge, the occlusal surface had a thickness of 2.0 mm or more, the axial surface had a thickness of 1.5 mm or more, the margin portion had a thickness of 0.8 mm or more, and the connecting portion had a height of 3.6 mm or more and a width of 5.2 mm or more. Bonding of the three-unit bridge to the base was performed as in Example 20. A cross-sectional schematic view illustrating the specimen of Comparative Example 8 bonded to the base is shown in FIG. 9.

Comparative Example 9 (Milled Frame + Commercial Resin)

As in Comparative Example 8, a composite material 9 was produced as in Reference Example 9 and cut into a block shape of 16 mm in height, 19 mm in width, and 40 mm in length. A milled frame was produced from the resulting block using a CAD/CAM system.

The commercial product B was built up on the milled frame, and polymerized and cured by performing photo irradiation for 90 seconds with a photopolymerization apparatus (LED Cure Master, manufactured by DENKEN-HIGHDENTAL Co., Ltd.). Then, the cured product was heat-treated at 110°C for 10 minutes in a heat polymerization apparatus, to produce a three-unit bridge. Bonding of the three-unit bridge to the base was performed as in Example 20.

Comparative Example 10 (Milled Frame Using Commercial Product + Commercial Resin)

A milled frame obtained by milling the commercial product A using a CAD/CAM system was used as the milled frame.

The commercial product B was built up on the milled frame to produce a three-unit bridge, as in Comparative Example 9. Bonding of the three-unit bridge to the base was performed as in Example 20.

Fracture strength was measured according to the method described above, for Examples 20 to 22 and Comparative Examples 8 to 10. The materials and dimensions, and results for each example or the like are shown in Table 5 below. The cross-sectional area of the second member (coating material) in Table 5 is the value obtained by subtracting the cross-sectional area of the first member (core material) from the cross-sectional area of the connecting portion of the three-unit bridge.

[Table 5]

| Table 5 | First Member (A) | | | | | | Second Member (B) | | Fracture Strength (N) | Method for Producing Three-Unit Bridge | |
| --- | --- | --- | --- | --- | --- | --- | --- | --- | --- | --- | --- |
| | Shape | Glass Fiber (D) | Presence of Pigment | Height (mm) | Width (mm) | Cross-Sectional Area (mm²) | Material | Cross-Sectional Area (mm²) | | Number of Milling Operations | Whether Resin Was Built Up or Not |
| Ex. 20 | Bar | (1) | Yes | 2 | 2 | 4 | Second Member Composition | 14.7 | 5226 | 1 | No |
| Ex. 21 | Bar | (2) | Yes | 2 | 2 | 4 | Second Member Composition | 14.7 | 5567 | 1 | No |
| Ex. 22 | Bar | (1) | Yes | 2.5 | 4 | 10 | Second Member Composition | 8.7 | 5910 | 1 | No |
| Comp. Ex. 8 | Frame (Prototype) | (2) | Yes | 2.5 | 4 | 10 | Second Member Composition | 8.7 | 2625 | 2 | No |
| Comp. Ex. 9 | Frame (Prototype) | (2) | Yes | 2.5 | 4 | 10 | Commercial Product B | 8.7 | 1789 | 1 | Yes |
| Comp. Ex. 10 | Frame (Commercial Product A) | Glass Fiber Woven Fabric | Yes | 2.5 | 4 | 10 | Commercial Product B | 8.7 | 1532 | 1 | Yes |

The three-unit bridges of Examples 20 to 22, produced using the composite material for dental milling of the present invention, had a fracture strength of 3000 N or more. By using the composite material for dental milling of the present invention, three-unit bridges having a fracture strength of 3000 N or more were produced by only a single milling operation.

The three-unit bridge of Comparative Example 8 had a fracture strength of less than 3000 N. The production of the three-unit bridge of Comparative Example 8 required two milling operations.

The three-unit bridges of Comparative Examples 10 and 11 had a fracture strength of less than 3000 N. For the three-unit bridges of Comparative Examples 9 and 10, the step of building up the second member (coating material) was required after the frame was produced by milling.

As in Examples 20 to 22, pre-forming the first member (core material) and the second member (coating material) into an integral piece during block molding allows a three-unit bridge to be produced by only a milling operation, without the step of manually forming the first member (core material) and the second member (coating material) into an integral piece and the step of building up the second member, which prevented the inclusion of air bubbles that may form a fracture initiation point. This is believed to be the reason why the high fracture strength was obtained in Examples 20 to 22.

The foregoing has shown that, by using the composite material for dental milling of the present invention (Examples 20 to 22), a three-unit bridge having a fracture strength of 3000 N or more can be produced by only a single milling operation, without performing the step of building up the second member. In Comparative Examples 8 to 10, three-unit bridges were not produced by a single milling operation, and had a fracture strength of 3000 N or less.

Industrial Applicability

Using the composite material for dental milling of the present invention, it is possible to produce a dental prosthesis, preferably a three-unit bridge, having a three-point bending strength required for a dental bridge and a three-point bending elastic modulus similar to those of tooth substances, and having a sufficient fracture strength, by a single milling operation using a CAD/CAM system.

Reference Signs List

1, 1': composite material for dental milling
2: first member (core material)
3: second member (coating material)
4, 4': dental prosthesis (three-unit bridge)
5: abutment portion
6: connecting portion
7: pontic portion
8: base
9: milled frame
10: milled resin teeth
11: stainless steel ball
12: jig for pressurization

**Claims**

1. A composite material for dental milling comprising (A) a first member and (B) a second member in contact with at least a portion of the first member (A),

   wherein the first member (A) comprises (C) a first curable resin and (D) a glass fiber,
   wherein the second member (B) comprises (E) a second curable resin and (F) an inorganic filler,
   wherein the first member (A) has a three-point bending strength of 500 MPa or more, and
   wherein the second member (B) has a three-point bending strength of less than 500 MPa.

2. The composite material for dental milling according to claim 1, wherein the second member (B) is coated or laminated on at least a portion of the first member (A).

3. The composite material for dental milling according to claim 1, wherein a ratio of a cross-sectional area of the second member (B) to a cross-sectional area of the first member (A) is 0.05 to 100.

4. The composite material for dental milling according to claim 1, wherein the cross-sectional area of the first member (A) is 3 to 330 $mm^2$.

5. The composite material for dental milling according to claim 1, wherein the cross-sectional area of the second member (B) is 270 to 330 $mm^2$.

6. The composite material for dental milling according to claim 1, wherein the first member (A) further comprises a coloring pigment.

7. The composite material for dental milling according to claim 1, wherein the composite material for dental milling has a three-point bending elastic modulus of 14 to 33 GPa.

8. A dental prosthesis produced by milling from the composite material for dental milling according to any one of claims 1 to 7.

9. The dental prosthesis according to claim 8, wherein the dental prosthesis is a bridge.

10. The dental prosthesis according to claim 9, wherein the bridge comprises an abutment portion, a connecting portion, and a pontic portion.

11. The dental prosthesis according to claim 10, wherein the dental prosthesis has a fracture strength of 3000 N or more.

FIG.1

FIG.2

FIG. 3a

FIG. 3b

FIG.3

1 2

4

1 1

8

FIG.4

FIG.5

FIG.6

FIG.7

FIG.8

FIG.9

## INTERNATIONAL SEARCH REPORT

| | International application No. |
|---|---|
| | **PCT/JP2024/014840** |

### A. CLASSIFICATION OF SUBJECT MATTER

***A61K 6/889***(2020.01)i; ***A61K 6/15***(2020.01)i; ***A61K 6/802***(2020.01)i; ***A61K 6/836***(2020.01)i; ***A61K 6/887***(2020.01)i
FI:    A61K6/889; A61K6/15; A61K6/887; A61K6/836; A61K6/802

According to International Patent Classification (IPC) or to both national classification and IPC

### B. FIELDS SEARCHED

Minimum documentation searched (classification system followed by classification symbols)

A61K6/889; A61K6/15; A61K6/802; A61K6/836; A61K6/887

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2002-104914 A (KURARAY CO., LTD.) 10 April 2002 (2002-04-10)<br>claims, paragraphs [0012]-[0024], [0033], [0041], examples | 1-11 |
| A | 豊田丈爾他. 各種硬質レジンの基礎的検討. 東北大学歯学雑誌. 1997, vol. 16, pp. 62-69, (TOYODA, Johji et al. Fundamental study of several hard resins. Tohoku Univ. Dent. J.)<br>table 1, fig. 6 | 1-11 |
| A | 中野浩輔他. コンポジットレジンによる支台築造の実験的研究. 日本補綴歯科学会雑誌. 1991, vol. 35, pp. 103-110, (NAKANO, Kosuke et al. Experimental Studies on Posts and Composite Cores. Annals of Japan Prosthodontic Society.)<br>p. 106, left column, first paragraph, fig. 4 | 1-11 |
| A | JP 2017-124981 A (KABUSHIKI KAISHA SHOFU) 20 July 2017 (2017-07-20)<br>entire text | 1-11 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

| | International application No. |
|---|---|
| | **PCT/JP2024/014840** |

| Patent document cited in search report | Publication date (day/month/year) | Patent family member(s) | Publication date (day/month/year) |
|---|---|---|---|
| JP 2002-104914 A | 10 April 2002 | (Family: none) | |
| JP 2017-124981 A | 20 July 2017 | US 2019/0125505 A1 | |
| | | EP 3192487 A1 | |

Form PCT/ISA/210 (patent family annex) (July 2022)

**REFERENCES CITED IN THE DESCRIPTION**

**Patent documents cited in the description**

- JP 2003104822 A
- JP 2017124981 A
- JP S50042696 A
- JP S56152408 A